# EUROPEAN PATENT SPECIFICATION

(11) **EP 2 957 913 B1**
(45) Date of publication and mention of the grant of the patent: **29.03.2017**
(21) Application number: 14712327.7
(22) Date of filing: 11.02.2014
(51) Int. Cl.: G01N 33/574

(54) **METHODS AND PRODUCTS FOR PROGNOSING THE CLINICAL EVOLUTION, OR PREDICTING THE RECURRENCE RISK, OF A PAPILLARY LESION OF THE BREAST**
VERFAHREN UND PRODUKTE ZUR PROGNOSE DER KLINISCHEN EVOLUTION ODER VORHERSAGE DES REZIDIVRISIKOS EINER PAPILLÄREN MAMMALÄSION
PROCÉDÉS ET PRODUITS POUR PRONOSTIQUER L'ÉVOLUTION CLINIQUE OU PRÉDIRE LE RISQUE DE RÉCIDIVE D'UNE LÉSION PAPILLAIRE DU SEIN

(30) Priority: 12.02.2013 ES 201330179
(43) Date of publication of application: 23.12.2015
(73) Proprietor: Oncomatryx Biopharma, S.L., 48160 Derio (Vizcaya) (ES)
(72) Inventor: FREIRE SALINAS, Francisco Javier, 39008 Santander - Cantabria (ES); GÓMEZ ROMAN, Javier, 39008 Santander - Cantabria (ES); DOMINGUEZ HORMAETXE, Saioa, 48160 Derio - Vizcaya (ES); SIMON BUELA, Laureano, 48160 Derio - Vizcaya (ES)
(74) Representative: ABG Patentes, S.L.
(86) International application number: PCT/ES2014/070094
(87) International publication number: WO 2014/125144

(56) References cited:
- WO-A2-2008/079269
- US-A1- 2013 023 434
- FREIRE J ET AL: "Collagen XIa1. A New Marker for Breast Carcinoma Malignancy in Core Needle Biopsies", MODERN PATHOLOGY, vol. 26, no. Suppl. 2, 1 February 2013 (2013-02-01), page 40A, XP002725735, & 102ND ANNUAL MEETING OF THE UNITED-STATES-AND-CANADIAN-ACADEMY-OF-PATH OLOGY (USCAP); BALTIMORE, MD, USA; MARCH 02 -08, 2013
- FREIRE J ET AL: "Collagen XIa1. A New Marker for Breast Carcinoma Malignancy in Core Needle Biopsies", LABORATORY INVESTIGATION, vol. 93, no. Suppl. 1, 30 January 2013 (2013-01-30), page 40A, XP002725736, & 102ND ANNUAL MEETING OF THE UNITED-STATES-AND-CANADIAN-ACADEMY-OF-PATH OLOGY (USCAP); BALTIMORE, MD, USA; MARCH 02 -08, 2013
- Javier Freire ET AL: "El pro-colágeno tipo XIa1", , 22 May 2013 (2013-05-22), XP055122155, Retrieved from the Internet: URL:https://www.seap.es/documents/228448/5 29737/05_Freire.pdf [retrieved on 2014-06-06]
- VARGAS A C ET AL: "The Role of Type XI and Type XVII Collagen in Breast Cancer Progression", MODERN PATHOLOGY, vol. 24, no. Suppl. 1, February 2011 (2011-02), page 67A, XP002725737, & 100TH ANNUAL MEETING UNITED STATES-AND-CANADIAN-ACADEMY-OF-PATHOLOGY; SAN ANTONIO, TX, USA; FEBRUARY 26 -MARCH 04, 2011
- KIM HOON ET AL: "Multi-cancer computational analysis reveals invasion-associated variant of desmoplastic reaction involving INHBA, THBS2 and COL11A1", BMC MEDICAL GENOMICS, vol. 3, no. 1, 3 November 2010 (2010-11-03), page 51, XP021082988,
- KAREN C HALSTED ET AL: "Collagen [alpha]1(XI) in normal and malignant breast tissue", MODERN PATHOLOGY, vol. 21, no. 10, 25 July 2008 (2008-07-25) , pages 1246-1254, XP055122756,
- GARCIA PRAVIA C ET AL: "Anti COL11, a new marker of infiltrating breast cancer", VIRCHOWS ARCHIV, vol. 455, no. Suppl. 1, 4 September 2009 (2009-09-04), pages 32-33, XP002695517,
- GARCIA PRAVIA C ET AL: "Anti-proCOL11A1, a new marker of infiltrating breast cancer", BRITISH JOURNAL OF SURGERY; THE 44TH CONGRESS OF THE EUROPEAN SOCIETY FOR SURGICAL RESEARCH TAKES PLACE THIS YEAR AT HÔTEL ATRIA, NIMES, FRANCE, 20 23 MAY 2009,, vol. 96, no. S5, 1 May 2009 (2009-05-01), page 11, XP002690550,
- JAVIER FREIRE ET AL: "Pro-COL11A1: A biomarker to predict malignant relapse of breast intraductal papillomas", JOURNAL OF CLINICAL ONCOLOGY AMERICAN SOCIETY OF CLINICAL ONCOLOGY, vol. 31, no. 2013, 20 March 2013 (2013-03-20), page 540, XP055122151,
- FREIRE J ET AL: "Collagen 11 Alpha 1 a Better Marker for Breast Tumor Invasiveness Than Myoepithelial Cell Markers", MODERN PATHOLOGY, vol. 27, no. Suppl. 2, 3 February 2014 (2014-02-03), page 49A, XP002725738, & 103RD ANNUAL MEETING OF THE UNITED-STATES-AND-CANADIAN-ACADEMY-OF-PATH OLOGY (USCAP); SAN DIEGO, CA, USA; MARCH 01 -07, 2014
- FREIRE J ET AL: "Collagen 11 Alpha 1 a Better Marker for Breast Tumor Invasiveness Than Myoepithelial Cell Markers", LABORATORY INVESTIGATION, vol. 94, no. Suppl. 1, 30 January 2014 (2014-01-30), page 49A, XP002725739, & 103RD ANNUAL MEETING OF THE UNITED-STATES-AND-CANADIAN-ACADEMY-OF-PATH OLOGY (USCAP); SAN DIEGO, CA, USA; MARCH 01 -07, 2014

## Description

### Field of the Invention

The present invention relates to *in vitro* methods and products for prognosticating clinical progression or predicting the risk and type of recurrence of a papillary lesion of the breast that are useful as a tool for selecting the treatment and follow-up that are most suited to each patient.

### Background of the Invention

Cancer is one of the main public health concerns worldwide. According to the GLOBOCAN database, administered by the International Agency for Research on Cancer belonging to the World Health Organization, over 10 million cases of cancer were diagnosed worldwide in the year 2000, and the number of deaths due to cancer in the same year was more than 6 million people.

During the process of becoming malignant, tumor cells change their gene expression pattern, which alters cell processes such as cell architecture maintenance, cell adhesion, cell death and cell proliferation. Not only do these modifications generate changes in the cells themselves, but they make some cells of the stroma receive altered molecular signals and change their pattern of behavior and their gene expression pattern, acquiring typical myofibroblast morphology. The molecules secreted by these myofibroblasts in response to the adjacent tumor can in turn contribute in a different manner to promoting tumor growth and invasion, such that a paracrine loop is set up between the tumor and stroma. In recent years, scientific evidence indicate, with increasingly more precision, that peritumoral stroma is one of the primary promoters of tumor invasiveness and of phenomena relating to resistance to therapy.

The methods and products for diagnosis claimed by the present inventors are comprised within this novel research framework because they are based on the use of the col11a1 gene and of the proCOL11A1 protein, present in invasive carcinoma stromal cells, as a potential marker for classifying intraductal papillomas of the breast according to their tendency to progress into a malignant tumor.

Breast cancer is the neoplasia with the highest incidence and mortality rates among women, therefore early diagnosis and treatment are vitally important. In recent years, with the emergence of genetics in medicine (particularly in medical oncology), various discoveries have been made which affect the prognosis and treatment of breast cancer, which have lead to reclassifying this pathology in terms of its genetic profile; the most significant discoveries have been about the epithelial component of these tumors, fundamentally epidermal growth factors (EGFR and Her-2), which have become therapeutic targets just like estrogen and progesterone receptors (Perou C., Sorlie T., Eisen M., 2000, Nature, 406:747-752; Nielsen T., Hsu F., Jensen K., 2004, Clin. Cancer Res, 10:5367-5374). Furthermore, a series of immunohistochemical markers which are very useful for differentiating between benign and malignant lesions in cases where conventional histochemical techniques are insufficient have been developed. There are benign sclerosing lesions in the breast, mainly sclerosing adenosis and radial scar which sometimes (due to their morphological pattern) cause difficulties in differential diagnosis with malignant infiltrating lesions (such as infiltrating ductal carcinoma and tubular carcinoma); this situation is even more complex in core needle biopsy, because since there is not a complete representation of the lesion its outlines cannot be seen, which is one of the keys to differential diagnosis, particularly when the basal myoepithelial cells present in benign lesions and absent in malignant infiltrating lesions cannot be recognized with standard staining.

There are immunohistochemical markers (such as p63, α-actin, smooth muscle myosin heavy chain, calponin, s100 protein or CD10) that stain the myoepithelial cells of the mammary ducts, helping to differentiate between such lesions. Among these markers, α-actin, smooth muscle myosin heavy chain and calponin are highly sensitive (89%) for basal myoepithelial cells; however, they are not specific for them and also stain smooth vascular muscle cells and stromal myofibroblasts; similar problems arise with s100 and CD10 (Lerwill, M.F., 2004, Am J Surg Path, 28:1076-1091). Werling *et al.* (Werling, R. W., Hwang, H., Yaziji, H., 2003, Am J Surg Path, 27:82-90) studied a series of cases corresponding to sclerosing adenosis, infiltrating ductal carcinoma, lobular carcinoma and ductal carcinoma *in situ,* analyzing the reactivity pattern of antibodies p63, smooth muscle myosin and calponin, demonstrating that all of them stained the myoepithelial cells of the benign and non-infiltrating malignant cases, in turn corroborating positivity for myosin and calponin in myofibroblasts and smooth vascular muscle cells. In that paper, p63 was the marker presenting the lowest cross-reactivity with stromal fibroblasts, without staining any of them, which revealed its high sensitivity and specificity for staining myoepithelial cells compared with the other antibodies studied; nevertheless, it did have several drawbacks, such as the sometimes discontinuous staining of the myoepithelial cells (particularly in carcinomas *in situ*) as well as focal positivity in up to 11% of tumor cell cases. On the other hand, Meryem *et al.* described that p63 was positive in 13 out of a total of 14 cases of metaplastic carcinoma of the breast (Meryem, Koker, M., Kleer, C.G., 2004, Am J Surg Path, 28:1506-1512). p63 is currently one of the most used markers for the differential diagnosis between benign sclerosing and malignant infiltrating lesions of the breast; however, up until now there was no stromal marker that helped differentiate between these lesions (Mattia Barbareschi, M., Pecciarini L., Cangi G., 2001, Am J Surg Path, 25:1054-1060).

Among lesions of the breast, those lesions having a papillary architecture (intraductal papilloma, papillomatosis, atypical papilloma and intraductal papillary carcinoma) are the most difficult to diagnose (Collins, L.C. and S.J. Schnitt, Histopathology, 2008, 52(1):20-9). Given their similarity, correct diagnosis of these lesions by means of morphology alone can be complicated, so pathologists resort to differential markers. The main indicator of the invasiveness of a papillary lesion is the presence or absence of myoepithelial cells (Ueng, S.H., et al. Arch Pathol Lab Med, 2009, 133(6):893-907) which can be seen by means of immunohistochemical staining of the markers discussed in the preceding paragraph (Mulligan, A.M. and F.P. O'Malley, Adv Anat Pathol, 2007, 14(2):108-19). Estrogen receptors (Mulligan & O'Malley mentioned above) or different cytokeratins such as CK5/6 and CK8 (Moumen, M., et al. Int J Dev Biol, 2011, 55(7-9):763-71) have also been used. However, although these techniques help in the diagnosis, there is not a clear consensus concerning the sensitivity and precision of these markers (Ueng, S.H., et al. Arch Pathol Lab Med, 2009, 133 (6) :893-907; Shouhed, D., et al. Am Surg, 2012, 78 (10) :1161-5) .

Of all the papillary lesions of the breast, intraductal papilloma is the most debated pathology in terms of diagnosis and treatment (Mulligan & O'Malley mentioned above). This disagreement is primarily because even though intraductal papilloma is a benign lesion *per se,* the association between intraductal papilloma and recurrence in the form of malignant pathologies is fairly high, reaching up to 33% if the initial lesion presents atypias (Ueng, S.H., et al. Arch Pathol Lab Med, 2009, 133 (6) 893-907; Ahmadiyeh, N. et al., Ann Surg Oncol, 2009, 16(8):2264-9; Valdes, E.K., et al. Ann Surg Oncol, 2006, 13(4):480-2; Rosen, Papilloma and related benign tumors, in Rosen's breast pathology, R. PP, Editor. 2009, Williams & Wilkins: Philadelphia. pp. 85-136).

In fact, there is enormous debate surrounding how to proceed when a new case is diagnosed as there are groups that advocate complete removal of the lesion in all cases (Rizzo, M., et al., Ann Surg Oncol, 2008, 15(4):1040-7) or only in cases in which atypias or multiple papillomas present (Agoff, S.N. and T.J. Lawton, Am J Clin Pathol, 2004, 122(3):440-3.; Rosen, E.L., et al., AJR Am J Roentgenol, 2002, 179(5):1185-92).

Correctly diagnosing papillomas having a presumably malignant behavior and benign papillomas is essential (Mulligan & O'Malley mentioned above; Choi, Y.D., et al., Acta Cytol, 2006, 50(1):35-40; Gomez-Aracil, V., et al., Cytopathology, 2002, 13 (1) :22-30) not only for the well-being of the patient because unnecessary interventions would be avoided, but rather due to economic repercussions. Markers such as CD44 (Tse, G.M., et al., J Clin Pathol, 2005, 58(11):1185-8) or cyclin D1 (Saddik, M., et al., Arch Pathol Lab Med, 1999, 123(2): 152-6) have been proposed as genes differentially expressed among different malignant and benign papillary lesions, but there is no correlation with the malignant recurrence of intraductal papillomas. Genetic alterations in the intraductal papillomas which are capable of predicting a higher susceptibility to malignant recurrence, such as the loss of heterozygosity (LOH) in chromosome 16 (Yoshida, M., et al., Virchows Arch, 2012, 460(5):497-504), have also been proposed in some publications. However, due to problems that are inherent to the technique itself, the absence of LOH does not imply a benign diagnosis.

Therefore, there continues to be a need to develop methods and products for predicting recurrences of papillary lesions of the breast, particularly, recurrences of intraductal papilloma of the breast, either in benign papillomas, or, advantageously, in recurrences with malignant progression to carcinomas. Said methods and products would be tools that are useful for prognosticating clinical progression and/or for predicting the risk of recurrence of said papillary lesion of the breast, as well as for selecting the treatment and follow-up that are most suited to each patient (subject diagnosed with papillary lesion of the breast) and/or for selecting a patient (subject diagnosed with papillary lesion of the breast) for a specific treatment. Said methods and products could additionally be used for classifying papillary lesions of the breast, specifically, intraductal papillomas of the breast, according to their risk of recurrence and becoming malignant.

Collagen is the main component of the extracellular matrix (ECM) . Proper expression of the genes encoding the different types of collagen is necessary for proper ECM assembly during embryonic development and for the maintenance thereof in the adult organism. Collagen XI (COL11) is a type of collagen that has been studied very little but which plays a fundamental role in regulating fibrillar networks in cartilaginous and non-cartilaginous matrices (Li, Y., et al., Cell, 1995, 80:423-430); these fiber networks are involved in different morphogenesis processes during embryonic development in vertebrates. Collagen alpha-1 (XI) chain (COL11A1) transcripts have been found during fetal development in cartilaginous tissues and also in other tissues such as bone, kidney, skin, muscle, tongue, intestine, liver, ear, brain and lung (Sandberg, J.M., et al., Biochem. J., 1993, 294:595-602; Yoshioka, H., et al., Dev. Dyn., 1995, 204:41-47). The extracellular matrix also plays an important role in certain biological processes, such as cell differentiation, proliferation and migration; therefore, deregulation of the expression of the genes encoding the proteins making up the extracellular matrix is associated with carcinogenic and metastatic processes (Boudreau, N., and Bissell, M.J., Curr. Opin. Cell Biol., 1998, 10:640-646; Stracke, M.L., et al., In vivo, 1994, 8:49-58). In the particular case of COL11A1, it has been demonstrated that stromal fibroblasts have elevated col11a1 gene expression levels in sporadic colorectal carcinomas, whereas this gene is not expressed in healthy colon (Fischer, H., et al., Carcinogenesis, 2001, 22:875-878). col11a1 gene expression has also been associated with pancreatic, breast, colon, lung, head and neck cancer (Kim, H. et al., BMC Medical Genomics, 2010, 3:51; Iacobuzio-Donahue, C., Am. J. Pathology, 2002, 160(4):1239-1249; Ellsworth, R.E., et al., Clin. Exp. Metastasis, 2009, 26: 205-13; Feng, Y., et al., Breast Cancer Res. Treat., 2007, 103(3):319-329; J.Gast. Liv. dis., 2008; Fischer, H., et al., BMC Cancer, 2001, 1:17-18; Fischer, H., et al., Carcinogenesis, 2001, 22:875-878; Suceveanu, A.I., et al., J. Gastrointestin. Liver Dis, 2009, 18(1):33-38; Chong, IW, et al., Oncol Rep, 2006, 16(5):981-988; Whan, K., Oncogene, 2002, 21:7598-7604; Schmalbach, C.E., et al., Arch. Otolaryngol. Head Neck Surg., 2004, 130(3):295-302) and bladder cancer (WO 2005/011619), and COL11A1 protein expression has been associated with pancreatic and colon cancer (Pilarsky, C., et al., J. Cel. Mol. Med., 2008, 12(6B):2823-35; Erkan, M., et al., Mol. Cancer, 2010, 9:88-103; Bowen, K.B., et al., J. Hist. Cyt., 2008, 56(3):275-283).

The use of proCOL11A1 protein as a differential marker between infiltrating squamous cell carcinoma of the head and neck and benign pathologies of the head and neck has recently been described (García-Ocaña et al., Poster "Immunohistochemical validation of procollagen COL11A1 as a desmoplastic tumor stroma marker", November 18, 2010, 3rd International Symposium of the IUOPA, Oviedo); as a differential marker between infiltrating ductal adenocarcinoma of the breast and sclerosing adenosis (Garcia Pravia et al., Abstract ESSR2009/218, "Anti-proCOL11A1, a new marker of infiltrating breast cancer", British J. Surgery, 2009, 96 (S5):11; García-Ocaña et al., Poster "Immunohistochemical validation of procollagen COL11A1 as a desmoplastic tumor stroma marker", November 18, 2010, mentioned above); and as a differential marker between pancreatic ductal adenocarcinoma and chronic pancreatitis (Garcia Pravia et al., Abstract ESSR2009/272, "ProCOL11A1 is an efficient marker of pancreatic cancer" British J. Surgery, 2009, 96 (S5):17; Garcia-Ocaña et al., Poster "Immunohistochemical validation of procollagen COL11A1 as a desmoplastic tumor stroma marker", November 18, 2010, mentioned above)).

A monoclonal antibody identified as 1E8.33 which specifically targets proCOL11A1 protein and is valid for the detection of said protein by means of immunohistochemistry has recently been developed (García-Ocaña, et al., Int J Oncol. 2012; 40(5):1447-54).

### Brief Description of the Invention

The present invention is defined by the appended claims. Any subject-matter identified in the application as "invention", "embodiment", "aspect", etc., which exceeds the scope of the invention as represented by the claims does not form part the claimed invention but only serves as background information to better understand the invention. The present inventors have discovered, by means of immunohistochemical analysis of intraductal papillomas of the breast using the monoclonal antibody 1E8.33 (García-Ocaña, et al., Int J Oncol. 2012; 40(5):1447-54), that proCOL11A1 protein is expressed in intraductal papillomas of the breast that have the capacity to show aggressive behavior, and it is not expressed in intraductal papillomas of the breast that show benign behavior.

This evidence makes said specific proCOL11A1 antibody a suitable antibody for the development of new *in vitro* methods and products for the diagnosis and/or prognosis of papillary lesions of the breast, more particularly for prognosticating clinical progression and predicting the risk of recurrence of a papillary lesion of the breast, such as an intraductal papilloma of the breast, on which to base the therapeutic decision for each patient diagnosed with a papillary lesion of the breast. Specifically, the invention provides methods and products for the highly sensitive and specific *in vitro* identification of those papillary lesions of the breast, particularly those intraductal papillomas of the breast, with the risk of progressing into potentially aggressive lesions and which are susceptible to being completely resected at the time of diagnosis and subjected to a subsequent and more comprehensive follow-up, and distinguishing them from the benign papillary lesions of the breast, particularly intraductal papillomas of the breast that are benign and do not require an aggressive therapeutic approach.

In one aspect, the invention relates to an *in vitro* method for prognosticating clinical progression and/or predicting the risk of recurrence of a papillary lesion of the breast, selected from method (A) and method (B), wherein
A) method (A) comprises
   - detecting the presence of proCOL11A1 protein in a sample from a subject diagnosed with a papillary lesion of the breast;
   wherein
   - detection of the presence of proCOL11A1 protein in said sample from a subject diagnosed with a papillary lesion of the breast is indicative that the risk of recurrence is high, or
   - non-detection of the presence of proCOL11A1 protein in said sample from a subject diagnosed with a papillary lesion of the breast is indicative that the risk of recurrence is low, and
B) method (B) comprises
   - comparing the proCOL11A1 protein expression level in said sample with said proCOL11A1 protein expression level in a control sample;
   wherein
   - a proCOL11A1 protein expression level in said sample from a subject diagnosed with a papillary lesion of the breast greater than said proCOL11A1 protein expression level in a control sample is indicative that the risk of recurrence is high, or
   - a proCOL11A1 protein expression level in said sample from a subject diagnosed with a papillary lesion of the breast equal to or less than said proCOL11A1 protein expression level in a control sample is indicative that the risk of recurrence is low.

In another aspect, the invention relates to a method for selecting a subject diagnosed with a papillary lesion of the breast for treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence, which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said subject is selected for said treatment:
- if the presence of proCOL11A1 protein is detected in said sample from the subject diagnosed with a papillary lesion of the breast; or
- if the proCOL11A1 protein expression level in said sample from the subject diagnosed with a papillary lesion of the breast is greater than said proCOL11A1 protein expression level in the control sample.

In another aspect, the invention relates to a method for selecting a subject diagnosed with a papillary lesion of the breast for treatment selected from treatment comprising the removal of said papillary lesion of the breast by conservative surgery and treatment comprising non-removal of said papillary lesion of the breast, which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said subject is selected for said treatment:
- if the presence of proCOL11A1 protein is not detected in said sample from the subject diagnosed with a papillary lesion of the breast; or
- if the proCOL11A1 protein expression level in said sample from the subject diagnosed with a papillary lesion of the breast is equal to or less than said proCOL11A1 protein expression level in the control sample.

In another aspect, the invention relates to a method for selecting treatment for a subject diagnosed with a papillary lesion of the breast, which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said treatment is selected from pharmacological treatment, treatment comprising surgical removal of said papillary lesion of the breast, and a combination thereof,
wherein said treatment is selected:
- if the presence of proCOL11A1 protein is detected in said sample from the subject diagnosed with a papillary lesion of the breast; or
- if the proCOL11A1 protein expression level in said sample from the subject diagnosed with a papillary lesion of the breast is greater than said proCOL11A1 protein expression level in the control sample.

In another aspect, the invention relates to the use of proCOL11A1 protein as a marker for prognosticating clinical progression or for predicting the risk of recurrence of a papillary lesion of the breast, or for selecting a subject diagnosed with a papillary lesion of the breast, or for selecting treatment for a subject diagnosed with a papillary lesion of the breast.

In another aspect, the invention relates to the use of a specific antibody recognizing proCOL11A1 protein as a marker for prognosticating clinical progression or for predicting the risk of recurrence of a papillary lesion of the breast, or for selecting a subject diagnosed with a papillary lesion of the breast, or for selecting treatment for a subject diagnosed with a papillary lesion of the breast.

In another aspect, the invention relates to the use of a kit comprising a reagent recognizing proCOL11A1 protein, or a reagent for detection and/or quantification of col11a1 gene expression, for prognosticating clinical progression or for predicting the risk of recurrence of a papillary lesion of the breast, or for selecting a subject diagnosed with a papillary lesion of the breast, or for selecting treatment for a subject diagnosed with a papillary lesion of the breast.

### Brief Description of the Drawings

Figure 1 shows the results of immunostaining for proCOL11A1 in a sample that is negative for intraductal papilloma of the breast (A), showing no recurrence, and in a sample that is positive for intraductal papilloma of the breast (B), showing recurrence in the form of infiltrating ductal carcinoma of the breast. Additional information can be found in Example 1.
Figure 2 is a bar graph showing the number of cases classified according to their immunolabeling for proCOL11A1. Additional information can be found in Example 1.

### Detailed Description of the Invention Definitions

To help understand the present patent application, the meanings of some of the terms and expressions as used in the context of the present invention are explained below.

The term "antibody" refers to a glycoprotein exhibiting binding activity specific for a particular protein which is called "antigen". The term "antibody" comprises monoclonal antibodies or polyclonal antibodies, which can be whole or fragments thereof; and it includes human antibodies, humanized antibodies and antibodies of non-human origin. "Monoclonal antibodies" are homogenous populations of highly specific antibodies which target a single site or an antigenic "determinant".

The term "cancer" or "carcinoma" refers to the disease characterized by uncontrolled proliferation of abnormal cells capable of invading adjacent tissues and spreading to distant organs.

The term "breast cancer" or "adenocarcinoma of the breast" refers to any malignant proliferative disorder of breast cells.

As it is used herein, the term "epitope" refers to an antigenic determinant of a protein, which is the amino acid sequence of the protein which a specific antibody recognizes.

The term "specificity" refers to the capability of detecting true negatives. 100% specificity means that there are no false positives (subjects who do not have the disease but get a positive result).

The term "clinical progression" generally refers to the progression of the clinical condition of the patient throughout the process of treating the disease, and, specifically in the sense used in this description, applied to a papillary lesion of the breast, it includes the assessment of the progression or recurrence of said papillary lesion of the breast into a malignant lesion, a carcinoma of the breast, or a benign lesion, and of the effect of the treatment, or the absence of treatment, applied to a subject with papillary lesion of the breast.

The term "gene" refers to a molecular chain of deoxyribonucleotides encoding a protein.

The term "col11a1 gene" refers to the gene encoding a collagen XI component called "pro-α1(XI) chain" which combines with two other collagen chains (pro-α2(XI) and pro-α1(II)) to form a procollagen molecule (proCOL11A1) that is enzymatically processed in the cells to form collagen XI fibers. Human col11a1 gene (also known as COLL6 or STL2), the reference gene sequence of which is NG_008033.1, takes up 150 kilobases (kb), contains 68 exons, is located in chromosome 1 (1p21) between base pairs 103342023 and 103574052, and encodes a 181 KDa protein consisting of 1806 amino acids (according to the isoform) which contains a peptide-signal (amino acids 1-36). This gene is conserved in humans, chimpanzees, cows, chickens, mice, rats and zebra fish. Mutations in this gene have been associated with Stickler II and Marshall syndromes. Polymorphisms of a nucleotide in this gene have been associated with susceptibility to suffer lumbar disc herniation. Several transcripts which mainly differ in the transcription of exon 6 variants, for example, one of 7.2 kb, encoding isoform A (NM_001854.3 (GI:98985806), NCBI database as of July 21, 2011) consisting of 1806 amino acids, another one of 7.3 kb, encoding isoform B (NM_080629.2 NCBI database as of July 21, 2011) consisting of 1806 amino acids (GI:98985810), another one of 6.9 kb, encoding isoform C (NM_080630.3 (GI:299523252), NCBI database as of July 21, 2011) consisting of 1690 amino acids (GI:299523253), another one of 7.2 kb, encoding isoform E (NM_001190709.1, NCBI database on August 1, 2011) consisting of 1767 amino acids (GI:299523257), etc., have been described. As it is used herein, the term "col11a1" refers not only to the human gene but also to the orthologs of other species. Low and homogenous col11a1 gene expression is observed in all the analyzed tissues except in adipocytes, where it is considerably more elevated (BioGPS: Gene Atlas U133A). In cell lines, elevated expression is detected in UASMC cells (umbilical artery smooth muscle cells), HN_NP cells (neuronal precursor cells), HN_Os cells (osteoblasts), Panc-1 cells (pancreatic cancer cells), H522 cells (lung cancer cells) and U251 cells (glioma cells) (RefExA), A-204 cells (heart rhabdomyosarcoma cells), SAOS-2 cells (bone marrow osteosarcoma cells) and SK-MEL28 cells (skin cancer cells) (GeneCards).

The terms "equal to" or "less than" applied to a protein expression level, specifically proCOL11A1 protein expression level, means that the amount or concentration of said protein in a specific sample is substantially the same as (equal to) or lower than (less than) that in another sample considered as the control sample or reference value; therefore, proCOL11A1 protein expression level in a sample from a subject diagnosed with a papillary lesion of the breast, such as an intraductal papilloma of the breast, without the tendency to progress into carcinoma (i.e., with a low risk of recurrence) is equal to (substantially the same as) or less than (lower than) said proCOL11A1 protein expression level in a control sample obtained from a control subject population without any history of breast tumors or of papillary lesions of the breast (e.g., intraductal papilloma of the breast) without the risk of recurrence in and/or of progression into carcinoma, or without any medical history of invasive carcinoma. In the context of the present invention, it is considered that a protein expression level, such as proCOL11A1 protein expression level, in the sample from the subject diagnosed with a papillary lesion of the breast is "equal to" said protein expression level in the control sample when said protein expression level in the sample from the subject is substantially the same as said protein expression level, for example, it is comprised between the reference value (proCOL11A1 protein expression level) plus/minus an amount of less than 3% of said reference value. Likewise, in the context of the present invention, it is considered that a protein expression level, such as proCOL11A1 protein expression level, in the sample from the subject diagnosed with a papillary lesion of the breast is "less" (or "lower") when said protein expression level in the sample from the subject decreases, for example, 3%, 5%, 10%, 25%, 50%, or even 100%, with respect to the reference value.

The term "papillary lesion of the breast" refers to a heterogeneous group of lesions of the breast having a papillary architecture. Illustrative, non-limiting examples of papillary lesions of the breast include intraductal papillomas, papillomatosis, atypical papilloma and intraductal papillary carcinomas. In a particular embodiment of the invention, said papillary lesion of the breast is an intraductal papilloma of the breast for all the inventive aspects considered.

The term "intraductal papilloma of the breast" refers to a papillomatous and therefore epithelial, villous, proliferation with a conjunctival-vascular axis, low mitotic activity, apocrine metaplasia stage and the absence of a cribriform pattern. Given its presentation, it can be solitary or single and multiple. It is a mass that forms in large ducts in the subareolar region, and it is a large mass when it is a single papilloma or a small mass when there are several together. It may be associated with hyperplastic alterations in the lobules.

As it is used herein, the term "predicting" applied to the risk of recurrence of a papillary lesion of the breast refers to the determination of the probability that a papillary lesion of the breast will progress into or recur (or not) in a malignant lesion in a subject diagnosed with said papillary lesion of the breast.

As it is used herein, the term "probability" measures the frequency with which a result (or set of results) is obtained when performing a random experiment for which all the possible results are known, under sufficiently stable conditions. The probability of obtaining a specific result can be "high", i.e., the frequency with which a result is obtained is greater than 50%, or "low", i.e., the frequency with which such result is obtained is less than 50%. According to the present invention, the probability that proCOL11A1 protein is detected in a sample from a subject diagnosed with a papillary lesion of the breast, or the probability that the proCOL11A1 protein expression level in a sample from a subject diagnosed with a papillary lesion of the breast is greater than proCOL11A1 protein expression level in a control sample, is higher in those cases in which said papillary lesion of the breast progresses into a malignant lesion, such as an invasive carcinoma, for example. As persons skilled in the art will understand, probability does not have to be 100% for all the evaluated subjects, although it preferably should be. However, within the context of the present invention, said term requires that a statistically significant part of the subjects diagnosed with a papillary lesion of the breast can be identified as subjects having a higher probability of obtaining a specific result, for example, that a papillary lesion of the breast will progress into or recur in a potentially malignant lesion or malignant recurrence. The person skilled in the art can determine whether or not an event is statistically significant, without major complications, using different known statistical evaluation tools, for example, by means of Student's t-test, Mann-Whitney test, determining confidence intervals, determining the p-value, etc. Additional information about these statistical tools can be found in Dowdy and Wearden, Statistics for Research. John Wiley & Sons, New York 1983. Preferred confidence intervals are at least 50%, at least 60%, at least 70%, at least 80%, at least 90%, or at least 95%. P-values are preferably 0.1, 0.05, 0.02, 0.01 or less.

The term "proCOL11A1" refers to the α1 chain of collagen XI, the processing of which gives rise to collagen XI α1 (COL11A1). proCOL11A1 has a triple-helix central domain with a rod-shaped structure flanked by non-collagenous propeptides at the N- and C-terminal ends; these propeptides can be eliminated by specific peptidases as they are secreted out of the cell, generating mature COL11A1, where α1 chains of collagen XI are assembled with one another and with α2 and/or α3 chains to form collagen XI fibers. proCOL11A1 is encoded by the col11a1 gene. Several human proCOL11A1 isoforms, for example, isoform A [NM_001854.3 (GI:98985806), NCBI database as of July 21, 2011] consisting of 1806 amino acids, isoform B [NP_542196.2 (GI:98985810), NCBI database as of July 21, 2011], isoform C [NM_080630.3 (GI:299523252), NCBI database as of July 21, 2011], etc., have been described. As it is used herein, the term "proCOL11A1" refers not only to human proCOL11A1 protein but also to orthologs of other species.

The col11a1 gene encodes a protein consisting of 1806 amino acids, having its triple helix region between amino acids 529 and 1542. It has two domains which are not always present in the mature protein, the C-terminal (amino acids 1564-1806) and the N-terminal (amino acids 37-511). It is part of type XI collagen which is formed in cartilage by three chains forming a triple helix, α1(XI), α2(XI) (encoded by the COL11A2 gene) and α3(XI) (generated by over-glycosylation of α1(II)) which can be substituted with α1(V). It is a component of the hyaline cartilage extracellular matrix, although it is also expressed in non-cartilaginous tissues and in tumor cell lines or in cell lines transformed by virus, but in this case the three chains of type XI collagen are not always co-expressed, which could mean that the fibers have a chain composition different from that of cartilage, which is homotrimeric or heterotypic in these areas (Yoshioka,H., J Biol Chem, 1990; 265(11):6423-6426; Lui, LCH, Biochem J, 1995; 311:511-516). It is believed to participate in fibrillogenesis, regulating the lateral growth of collagen II fibers, serving as a support for said fibers, and being located inside the formed fiber (Weis, MA., J Biol Chem 2010; 285(4):2580-2590). It is synthesized as procollagen, which is proteolytically processed after secretion, terminal peptides N (37-511) and C (1564-1806) being eliminated (Halsted, KC., Mod Pathol 2008; 21(10):1246-1254). An Npp (amino propeptide) or TSP region (38-229), which is also found in 7 other types of collagens, in laminin and in thrombospondin, is contained within the amino-terminal peptide (NTD) and contains a processing site for BMP-1 (Warner, L., J Biol Chem 2006; 281(51):39507-39515, Gregory, KE., J Biol Chem 2000; 275(15): 11498-11506). In the case of α1(XI), this region is not always eliminated, sometimes remaining exposed on the surface of collagen fibers for a long time (Fallahi, A., Prot Sci 2005; 14:1526-1537). This region is very similar to the LNS domains, having potential binding sites for heparin and calcium, which could mean cell-ME communication activity by binding to heparan sulfate proteoglycans (Warner, L., J Biol Chem 2006; 281(51):39507-39515, Fallahi, A., Prot Sci 2005; 14:1526-1537), even after being proteolyzed from the helical domain. Furthermore, NTD covers a variable region, having different sequences and characteristics according to alternative splicing, combining exons 6-7-8 of the gene. These variants have tissue and temporal specificity (Warner, L., J Biol Chem 2006; 281 (51) :39507-39515), and they affect the Npp processing time.

In the sense used herein, the expression "prognosticating clinical progression" of a papillary lesion of the breast generally refers to predicting the progression of the clinical condition of the papillary lesion of the breast throughout the process of treating the disease, and, particularly, if it will recur in a carcinoma of the breast. By way of illustration, if the presence of proCOL11A1 protein is detected in the sample from the subject diagnosed with a papillary lesion of the breast or if the proCOL11A1 protein expression level in said sample is greater than said proCOL11A1 protein expression level in a control sample, then the prognosis of clinical progression will be recurrence in carcinoma of the breast. Furthermore, if the presence of proCOL11A1 protein is not detected in the sample from the subject diagnosed with a papillary lesion of the breast or if the proCOL11A1 protein expression level in said sample is less than said proCOL11A1 protein expression level in a control sample, then the prognosis of clinical progression will be no recurrence in carcinoma of the breast.

The term "protein" refers to a molecular amino acid chain with biological activity. The term includes all forms of post-translational modifications, for example glycosylation, phosphorylation or acetylation. The terms "protein", "peptide" and "polypeptide" are used interchangeably in this description.

The term "recurrence" generally refers to the reoccurrence of the lesion after a relatively long period of absence of disease. In the sense used herein, the term "recurrence" applied to a papillary lesion of the breast particularly refers to the reoccurrence of a papillary lesion of the breast in the form of a malignant lesion, such as in the form of a carcinoma of the breast or breast tumor, ductal adenocarcinoma, ductal carcinoma *in situ,* invasive ductal carcinoma, lobular carcinoma, lobular carcinoma *in situ,* invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer.

As it is used herein, the term "risk" refers to the relative risk or probability that a specific papillary lesion of the breast will progress into a potentially malignant lesion such as, for example, a carcinoma of the breast or breast tumor, ductal adenocarcinoma, ductal carcinoma *in situ,* invasive ductal carcinoma, lobular carcinoma, lobular carcinoma *in situ,* invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer. The risk can be "high" or "elevated", or, alternatively, "low" or "reduced". The risk can be determined without major complications by a person skilled in the art, using different known statistical evaluation tools according to the information obtained about the detection of the presence of proCOL11A1 protein or of the proCOL11A1 protein expression level in a sample from a subject diagnosed with a papillary lesion of the breast.

The term "sensitivity" refers to the detection of true positives (e.g., positive diagnosis of a pathology when the patient has said pathology); a 100% sensitivity means that there are no false negatives (negative diagnosis in patients that have said pathology).

The term "subject" or "individual" refers to a member of a mammalian species and includes, but is not limited to, domestic animals, primates and humans; the subject is preferably a male or female human being of any age or race.

The term "greater than" applied to a protein expression level, specifically proCOL11A1 protein expression level, means that the amount or concentration of said protein in a specific sample is higher than in another sample considered as the control sample or reference value; therefore, the proCOL11A1 protein expression level in a sample from a subject diagnosed with a papillary lesion of the breast, such as an intraductal papilloma of the breast, with the tendency to progress into a carcinoma (i.e., with the risk of malignant recurrence) is higher (greater) than said proCOL11A1 protein expression level in a control sample obtained from a control subject population without any history of breast tumors or papillary lesions of the breast (e.g., intraductal papilloma of the breast) without the risk of recurrence in and/or of progression into carcinoma, or without any medical history of invasive carcinoma. In the context of the present invention, it is considered that a protein expression level, such as the proCOL11A1 protein expression level, in the sample from the subject diagnosed with a papillary lesion of the breast, is "greater than" (or "higher than") said protein expression level in a control sample (reference value) when said protein expression level in the sample from the subject increases, for example, 3%, 5%, 10%, 25%, 50%, 100% or even more when compared with the reference value for said protein in the control sample, or when it increases, for example, at least 1.1-fold, 1.5-fold, 2-fold, 5-fold, 10-fold, 20-fold, 30-fold, 40-fold, 50-fold, 60-fold, 70-fold, 80-fold, 90-fold, 100-fold, or even more when compared with the reference value for said protein.

As it is used herein, the term "treatment" generally refers to the application of a therapy to alleviate or eliminate a pathology or to reduce or eliminate one or more symptoms associated with said pathology. Said therapy can include surgical intervention, pharmacological treatment, radiation therapy treatment, etc.

The term "tumor" refers to any abnormal mass of tissue resulting from a benign (non-cancerous) or malignant (cancerous) neoplastic process.

As it is used herein, the term "variant" applied to heavy and light chain sequences of an antibody refers to substantially similar sequences. From the qualitative viewpoint, the variants generally have the same biological activity as the native sequence. A polypeptide sequence variant can be a polypeptide sequence derivative comprising the addition, deletion or substitution of one or more amino acids present in the native sequence. Antibody heavy and light chain sequence variants can differ from the sequences described in the framework regions or in the complementarity determining regions or CDRs of any of the heavy or light chains. By way of illustration, the term "variant" applied to monoclonal antibody 1E8.33 (García-Ocaña, et al., Int J Oncol. 2012; 40(5):1447-54) includes amino acid sequences having at least about 70%, 75%, 80%, 85%, 90%, 91%, 92%, 93%, 94%, 95%, 96%, 97%, 98% or 99% sequence identity with any of the amino acid sequences of the CDRs comprised in the heavy chain variable region or the light chain variable region of said monoclonal antibody 1E8.33, provided that the resulting variant maintains the biological activity of the native sequence, i.e., the antibody variant maintains the capacity to specifically recognize proCOL11A1 protein.

### Methods for the prognosis of clinical progression/prediction of risk

The present invention is based on the discovery that proCOL11A1 protein expression is increased in intraductal papillomas of the breast, a papillary lesion of the breast that is representative of this group of lesions which have the tendency to progress into carcinoma of the breast.

Therefore, in one aspect the invention relates to an *in vitro* method for prognosticating clinical progression and/or predicting the risk of recurrence of a papillary lesion of the breast, hereinafter "first method of the invention", selected from method (A) and method (B), wherein
A) method (A) comprises
   - detecting the presence of proCOL11A1 protein in a sample from a subject diagnosed with a papillary lesion of the breast;
   wherein
   - detection of the presence of proCOL11A1 protein in said sample from a subject diagnosed with a papillary lesion of the breast is indicative that the risk of recurrence is high, or
   - non-detection of the presence of proCOL11A1 protein in said sample from a subject diagnosed with a papillary lesion of the breast is indicative that the risk of recurrence is low, and
B) method (B) comprises
   - comparing the proCOL11A1 protein expression level in said sample with said proCOL11A1 protein expression level in a control sample;
   wherein
   - a proCOL11A1 protein expression level in said sample from a subject diagnosed with a papillary lesion of the breast greater than said proCOL11A1 protein expression level in a control sample is indicative that the risk of recurrence is high, or
   - a proCOL11A1 protein expression level in said sample from a subject diagnosed with a papillary lesion of the breast equal to or less than said proCOL11A1 protein expression level in a control sample is indicative that the risk of recurrence is low.

The first method of the invention is a highly sensitive and specific method, and it is based on the fact that subjects or individuals diagnosed with a papillary lesion of the breast, such as an intraductal papilloma of the breast, for example, with the tendency to recur or progress into carcinoma, express and show proCOL11A1 or have elevated proCOL11A1 levels in absolute terms or compared with the corresponding levels s in control samples from subjects without any medical history of breast tumors or with papillary lesions of the breast, for example, intraductal papillomas of the breast, without the risk of recurrence, or with other benign lesions of the breast.

To put the any of the alternatives [Method (A) or (Method (B)] of the first method of the invention into practice, a sample, such as a biological sample, is obtained from the individual to be studied. Illustrative, non-limiting examples of said sample include a core of tissue obtained by means of core needle biopsy (CNB), a piece of breast tissue obtained through surgery, for example, by means of biopsy, cytology or surgical resection, as well as a biological fluid, such as blood, serum, nipple aspirate fluid, urine, etc. Depending on the type of samples, to simplify their storage and handling they can be fixed in formol and embedded in paraffin, or, alternatively, they can be first frozen and then embedded in a cryosolidifiable medium.

The samples to be analyzed can be obtained from subjects previously diagnosed with a papillary lesion of the breast, or non-diagnosed subjects. In a particular embodiment, said papillary lesion of the breast is an intraductal papilloma of the breast.

### Method (A)

In a particular embodiment, the first method of the invention comprises detecting the presence of proCOL11A1 protein in a sample from a subject diagnosed with a papillary lesion of the breast [Method (A)], wherein detection of the presence of proCOL11A1 protein in said sample from a subject diagnosed with a papillary lesion of the breast is indicative that the risk of recurrence is high, or wherein non-detection of the presence of proCOL11A1 protein in said sample from a subject diagnosed with a papillary lesion of the breast is indicative that the risk of recurrence is low.

The presence of proCOL11A1 in the sample to be analyzed can be detected by means of any conventional method which allows detecting the presence of a protein, specifically proCOL11A1, in a sample. In a particular embodiment, said sample is a breast tissue sample. Virtually any conventional method for detection of the presence of a protein in a tissue sample can be used in the framework of the invention for detecting the presence of proCOL11A1. Illustrative, non-limiting examples of said methods include methods based on the use of antibodies, affinity chromatography techniques, ligand binding assays, etc.

In a particular embodiment, detection of the presence of proCOL11A1 is performed by means of an immunoassay based on the formation of an antigen-antibody type complex, by means of using one (or more antibodies) recognizing one (or more) proCOL11A1 epitopes, and subsequent viewing of the complexes formed by any suitable technique, including both radioactive and non-radioactive techniques such as, for example, colorimetric, fluorometric, luminescent techniques (e.g., bioluminescent techniques, chemiluminescent techniques, etc.), etc., using to that end, where appropriate, secondary antibodies labeled with the suitable markers.

The antibodies to be used for putting this particular embodiment of the first method of the invention [Method (A)] into practice are specific antibodies recognizing proCOL11A1 protein, or an antigenic fragment of proCOL11A1, i.e., antibodies recognizing a proCOL11A1 epitope. The antibodies that can be used in such assays can be polyclonal sera, monoclonal antibodies, antibody fragments, such as Fv, Fab, Fab' and F(ab')2, scFv (single chain Fv), diabodies, triabodies, tetrabodies, combibodies, etc., which are capable of recognizing and binding to proCOL11A1 or to an antigenic fragment thereof; said antibodies can be human antibodies, humanized antibodies or antibodies of non-human origin. The antibody recognizing proCOL11A1 protein is advantageously an antibody specifically recognizing the VAR subdomain in the N-terminal propeptide of proCOL11A1. Illustrative examples of antibodies specifically detecting proCOL11A1 are mentioned in international patent application PCT/ES2012/070616 [WO 2013/021088]. In a particular embodiment, said antibody is monoclonal antibody 1E8.33 (Garcia-Ocaña, et al., Int J Oncol. 2012; 40(5):1447-54), an antibody specifically recognizing proCOL11A1 protein, without detecting other proteins with a high sequence homology, such as COL5A1 protein.

The antibody recognizing proCOL11A1 can optionally be conjugated to a carrier. Said antibody recognizing proCOL11A1 can also be labeled or not; illustrative, non-limiting examples of markers that can be used include radioactive isotopes, enzymes, fluorophores, chemiluminescent reagents, enzyme substrates or co-factors, enzyme inhibitors, particles, dyes, etc. The unlabeled antibodies can be used in binding assays, whereas the labeled antibodies can be used in a wide range of assays.

There is a wide range of assays that are well-known by persons skilled in the art which can be used for putting this particular embodiment of the first method of the invention [Method (A)] into practice, said assays using unlabeled antibodies (primary antibody) recognizing proCOL11A1 and labeled antibodies (secondary antibodies); Western-blot or immunoblot, ELISA (enzyme-linked immunosorbent assay), RIA (radioimmunoassay), competitive EIA (enzyme immunoassay), DAS-ELISA (double antibody sandwich ELISA), immunohistochemical assays, immunohistochemical assays, multiplex detection techniques based on the use of protein microspheres, biochips or microarrays including specific antibodies, or assays based on colloidal precipitation, etc., are included among these techniques.

In a particular preferred embodiment, detection of the presence of proCOL11A1 according to method (A) is carried out by means of an immunohistochemical assay, a known histopathological method based on the use of a specific antibody, usually labeled with a marker (for example, an enzyme), which can transform a substrate into a visible compound without affecting the antibody's capacity to form a complex with the antigen (proCOL11A1 in this case), applied to an organic tissue sample. With the use of one of the specific techniques (peroxidase, antiperoxidase, fluorescein, etc.), the antigen-antibody complex formed can be located and identified in the tissue or cytological samples to be studied, so the characteristic antigenic markers of different cells are identified and the type of cell involved in the sample can be determined.

Briefly, in a particular embodiment of method (A), the sample which can be a fresh sample, a frozen sample or a sample embedded in paraffin and fixed using a protective agent (e.g., formol or the like) comprising breast tissue to be analyzed for the purpose of detecting the presence of proCOL11A1 by means of an immunohistochemical assay, is stained with an antibody specific for proCOL11A1, and the frequency of cells that have been stained and the staining intensity are determined. If needed, the sample to be analyzed is subjected to conditioning treatment which can include one or more of the following processes: fixing the tissue, obtaining suitably thick sections, recovering the antigen, blocking molecules that may interfere in the indirect reaction, preventing non-specific binding, etc. Detection of proCOL11A1 by means of said immunohistochemical assay in the sample to be analyzed is advantageously carried out in parallel with tissue or cytological samples used as a positive marker and as a negative marker, and if desired, healthy tissues from the same origin as that of the papillary lesion of the breast being analyzed can be used as a reference. It is also common to use a background control. The sample is typically assigned a value indicative of total expression which is calculated according to the frequency of stained cells and the intensity in each of the stained cells. The typical criteria for assigning expression values to samples are described, for example, in Handbook of Immunohistochemistry and In situ Hybridization in Human Carcinomas, M. Hayat Ed., 2004, Academic Press.

Example 1 describes an immunohistochemical assay for detecting the presence of proCOL11A1 in which the previously conditioned sample comprising breast tissue to be analyzed is contacted with the monoclonal antibody against proCOL11A1, and when the incubation period ends, it is developed with diaminobenzidine (DAB) using a suitable detection system.

Detection of the presence of proCOL11A1 by means of an immunohistochemical assay has a number of advantages because it is widely used in anatomic pathology laboratories, which enables being able to immediately apply the first method of the invention, particularly method (A), by means of said technique, and furthermore it is a highly automated technique, which facilitates performing it under the same conditions.

After detection of the presence of proCOL11A1 in a sample from a subject diagnosed with a papillary lesion of the breast, it is possible to relate detection of the presence (or non-detection of the presence - absence) of proCOL11A1 in said sample with the prognosis of clinical progression of said papillary lesion or with the prediction of risk of recurrence of said papillary lesion of the breast. In a particular embodiment, said papillary lesion of the breast is an intraductal papilloma of the breast.

Therefore in a subsequent step, method (A) involves correlating detection of the presence (or non-detection of the presence - absence) of proCOL11A1 with a prognosis of clinical progression of said papillary lesion or with the prediction of risk of recurrence of said papillary lesion of the breast.

This correlation can indicate that:
- the prognosis of clinical progression of said papillary lesion of the breast is recurrence in carcinoma of the breast when the presence of proCOL11A1 protein is detected in the sample from the subject diagnosed with a papillary lesion of the breast under study; or that
- the prognosis of clinical progression of said papillary lesion of the breast is no recurrence in carcinoma of the breast when the presence of proCOL11A1 protein is not detected in the sample from the subject diagnosed with a papillary lesion of the breast under study.

This correlation can alternatively indicate that:
- the risk of recurrence of the papillary lesion of the breast is high when the presence of proCOL11A1 protein is detected in the sample from the subject diagnosed with a papillary lesion of the breast under study; or that
- the risk of recurrence of the papillary lesion of the breast is low when the presence of proCOL11A1 protein is not detected in the sample from the subject diagnosed with a papillary lesion of the breast under study.

This information can be used efficiently by a specialist for selecting the most suitable treatment to be administered to the subject diagnosed with a papillary lesion of the breast according to the risk of recurrence of said papillary lesion of the breast.

In the sense used herein, the expression "detection of the presence of proCOL11A1", or the like, refers to the capacity to detect proCOL11A1 in the analyzed sample using any of the methods referred to above. Likewise, in the sense used herein, the expression "non-detection of the presence of proCOL11A1", or the like, refers to the incapacity to detect proCOL11A1 in the analyzed sample using any of the methods referred to above.

### Method (B)

In another particular embodiment, the first method of the invention comprises comparing the proCOL11A1 protein expression level in the sample from a subject diagnosed with a papillary lesion of the breast with said proCOL11A1 protein expression level in a control sample, wherein a proCOL11A1 protein expression level in said sample from the subject diagnosed with a papillary lesion of the breast greater than said proCOL11A1 protein expression level in a control sample is indicative that the risk of recurrence is high, or wherein a proCOL11A1 protein expression level in said sample from the subject diagnosed with a papillary lesion of the breast equal to or less than said proCOL11A1 protein expression level in a control sample is indicative that the risk of recurrence is low. In a particular embodiment, said papillary lesion of the breast is an intraductal papilloma of the breast.

The characteristics of the sample to be analyzed have already been mentioned above.

Method (B) comprises determining the proCOL11A1 protein expression level in a sample from a subject diagnosed with a papillary lesion of the breast and comparing it with said proCOL11A1 protein expression level in a control sample.

In a particular embodiment, the determination of the proCOL11A1 protein expression level is carried out by means of quantification or determination of the amount or concentration of said proCOL11A1 protein in said sample.

The amount or concentration of proCOL11A1 protein present in said samples can be quantified by means of any conventional method which allows detecting and quantifying said protein in the samples to be analyzed. In this case, method (B) comprises performing an extraction step for obtaining a protein extract containing said protein. The protein extracts can be obtained by means of conventional methods (Chomczynski et al., Anal. Biochem., 1987, 162:156; Chomczynski P., Biotechniques, 1993, 15:532).

Virtually any duly adapted conventional method for determining the amount or concentration of proteins in a sample can be used in the framework of the present invention for quantifying the amount or concentration of proCOL11A1 protein present in a sample. By way of non-limiting illustration, the amount or concentration of said proCOL11A1 protein can be determined by means of conventional methods, for example, by means of using antibodies with proCOL11A1-binding capacity and subsequently quantifying the complexes formed. In a particular embodiment, method (B) comprises contacting the protein extract of the sample with a composition comprising one or more specific antibodies against one or more epitopes of the proCOL11A1 protein, under conditions that allow forming antibody:proCOL11A1 complexes, and determining the amount or concentration of proCOL11A1 protein present in the sample. Illustrative, non-limiting examples of said methods include methods based on the use of antibodies, affinity chromatography techniques, ligand binding assays, etc.

In a particular embodiment, detection of the presence of proCOL11A1 is performed by means of an immunoassay based on the formation of an antigen-antibody type complex, by means of using one (or more antibodies) recognizing one (or more) proCOL11A1 epitopes, and subsequent viewing and quantification of the complexes formed by any suitable technique, including both radioactive and non-radioactive techniques such as, for example, colorimetric, fluorometric, luminescent (e.g., bioluminescent, chemiluminescent, etc.) techniques, etc., using to that end, where appropriate, secondary antibodies labeled with the suitable markers.

The antibodies to be used for putting this particular embodiment of the first method of the invention [Method (B)] into practice are specific antibodies recognizing proCOL11A1 protein, or an antigenic fragment of proCOL11A1, i.e., antibodies recognizing a proCOL11A1 epitope. The antibodies that can be used in such assays can be polyclonal sera, monoclonal antibodies, antibody fragments, such as Fv, Fab, Fab' and F(ab')2, scFv (single chain Fv), diabodies, triabodies, tetrabodies, combibodies, etc., which are capable of recognizing and binding to proCOL11A1 or to an antigenic fragment thereof; said antibodies can be human antibodies, humanized antibodies or antibodies of non-human origin. The antibody recognizing proCOL11A1 protein is advantageously an antibody specifically recognizing the VAR subdomain in the N-terminal propeptide of proCOL11A1. Illustrative examples of antibodies specifically detecting proCOL11A1 are mentioned in relation to method (A), the content of which is incorporated herein by reference. International patent application PCT/ES2012/070616 [WO 2013/021088] describes antibodies specifically recognizing the VAR subdomain in the N-terminal propeptide of proCOL11A1. In a particular embodiment, said antibody is monoclonal antibody 1E8.33 (García-Ocaña, et al., Int J Oncol. 2012; 40(5):1447-54).

The antibody recognizing proCOL11A1 can be optionally conjugated to a carrier. Said antibody recognizing proCOL11A1 can also be labeled or not; illustrative, non-limiting examples of markers that can be used include radioactive isotopes, enzymes, fluorophores, chemiluminescent reagents, enzyme substrates or co-factors, enzyme inhibitors, particles, dyes, etc. The unlabeled antibodies can be used in binding assays, whereas the labeled antibodies can be used in a wide range of assays.

There is a wide range of assays that are well-known by persons skilled in the art which can be used for putting this particular embodiment [Method (B)] of the first method of the invention into practice, said assays using unlabeled antibodies (primary antibody) recognizing proCOL11A1 and labeled antibodies (secondary antibodies); Western-blot or immunoblot, ELISA, RIA, competitive EIA, DAS-ELISA, immunohistochemical assays, immunohistochemical assays, multiplex detection techniques based on the use of protein microspheres, biochips or microarrays including specific antibodies, or assays based on colloidal precipitation, etc., are included among these techniques.

In a particular preferred embodiment, detection of the presence of proCOL11A1 according to method (B) is carried out by means of an enzyme-linked immunosorbent assay (ELISA), a known immunodetection method which is based on the use of one or two specific antibodies, one of them usually labeled with an enzyme, which can transform a substrate into a visible compound without affecting the antibody's capacity to form a complex with the antigen (proCOL11A1 in this case), applied to a protein extract generally coming from a biological fluid (e.g., blood, serum, urine, nipple aspirate fluid, etc.) or from a portion of tissue, or applied to the biological fluid itself. With the use of any of the specific techniques (peroxidase, antiperoxidase, fluorescein, etc.), the antigen-antibody complex formed can be detected and quantified in the samples to be studied, the protein levels to be analyzed in samples of a different origin being able to be easily quantified.

Briefly, in a particular embodiment of method (B), the sample which can be a fresh sample or a frozen blood sample, serum sample, urine sample, nipple aspirate fluid sample, etc., comprising a biological fluid for the purpose of detecting the presence of proCOL11A1 by means of an immunohistochemical assay, is contacted with labeled anti-pro-COL11A1 antibody/antibodies and the concentration of the protein is quantified according to the intensity of the enzymatic reaction associated with the labeling of the antibody. If needed, the sample to be analyzed is subjected to a protein extraction process for the purpose of concentrating said proteins, facilitating their detection by means of conventional methods (Chomczynski et al., Anal. Biochem., 1987, 162:156; Chomczynski P., Biotechniques, 1993, 15:532). Detection of proCOL11A1 in the sample to be analyzed by means of said immunohistochemical assay is advantageously carried out in parallel with samples containing known proCOL11A1 levels as a reference (standard curve), consisting of human samples of a known concentration, recombinant proCOL11A1 protein or cell line extracts expressing the protein, which will allow calculating the concentration of proCOL11A in the samples to be analyzed by comparing the intensities obtained.

Detection of the presence of proCOL11A1 by means of an ELISA-type immunodetection assay has a number of advantages because it is widely used in biochemistry laboratories, which enables being able to immediately apply the first method of the invention, particularly method (B), by means of said technique, and furthermore it is a highly automated technique, which facilitates performing it under the same conditions.

Method (B) comprises the step of comparing the amount or concentration of proCOL11A1 protein determined in the sample from the subject diagnosed with a papillary lesion of the breast object of study with the amount or concentration of proCOL11A1 protein of the control sample (reference value). In a particular embodiment, the control sample is a sample from subjects without any medical history of breast tumors or of papillary lesions of the breast (e.g., intraductal papilloma of the breast) without the risk of recurrence in and/or of progression into carcinoma.

Therefore, once the reference value has been established, the proCOL11A1 protein expression level in the sample from the subject diagnosed with a papillary lesion of the breast under study is compared with the reference value. As a result of this comparison, the proCOL11A1 protein expression level in the sample from the subject can be "greater than" (higher than), "less than" ("lower than") or "equal to" said reference value for said protein in the control sample. As discussed above, in the context of the present invention, it is considered that the protein of interest (proCOL11A1) expression level in the sample from the subject under study is "greater than" the reference value for said protein when said protein expression level in the sample from the subject under study increases, for example, 3%, 5%, 10%, 25%, 50%, 100% or even more when compared with the reference value for said protein, or when it increases, for example, at least 1.1-fold, 1.5-fold, 2-fold, 5-fold, 10-fold, 20-fold, 30-fold, 40-fold, 50-fold, 60-fold, 70-fold, 80-fold, 90-fold, 100-fold or even more when compared with the reference value for said protein. Likewise, in the context of the present invention, it is considered that an expression level of the gene of interest in the sample from the subject is "less than" the reference value for said protein when said protein expression level in the sample from the subject under study decreases, for example, 3%, 5%, 10%, 25%, 50%, 75%, or even 100% when compared with the reference value for said protein. Additionally, in the context of the present invention, it is considered that an expression level of the gene of interest in the sample from the subject is equal to" the reference value for said protein when said protein expression level in the sample from the subject under study is substantially the same as the reference value for said protein (i.e., the reference value ± 3%).

Additionally, in a subsequent step, method (B) comprises the step of correlating the result obtained after comparing the amount or concentration of proCOL11A1 protein in the sample from a subject diagnosed with a papillary lesion of the breast with the amount or concentration of said proCOL11A1 protein in a control sample with the prognosis of clinical progression of said papillary lesion or with the prediction of risk of recurrence of said papillary lesion of the breast. In a particular embodiment, said papillary lesion of the breast is an intraductal papilloma of the breast.

This correlation can indicate that:
- the prognosis of clinical progression of said papillary lesion of the breast is recurrence in carcinoma of the breast when the amount or concentration of proCOL11A1 protein in the sample from the subject diagnosed with a papillary lesion of the breast is greater than the amount or concentration of said proCOL11A1 protein in the control sample, or that
- the prognosis of clinical progression of said papillary lesion of the breast is no recurrence in carcinoma of the breast when the amount or concentration of proCOL11A1 protein in the sample from the subject diagnosed with a papillary lesion of the breast is less than the amount or concentration of said proCOL11A1 protein in the control sample.

This correlation can alternatively indicate that:
- the risk of recurrence of the papillary lesion of the breast is high when the amount or concentration of proCOL11A1 protein in the sample from the subject diagnosed with a papillary lesion of the breast is greater than the amount or concentration of said proCOL11A1 protein in the control sample, or that
- the risk of recurrence of the papillary lesion of the breast is low when the amount or concentration of proCOL11A1 protein in the sample from the subject diagnosed with a papillary lesion of the breast is equal to or less than the amount or concentration of said proCOL11A1 protein in the control sample.

In another particular embodiment of method (B), the determination of the proCOL11A1 protein expression level is carried out by means of quantification of the col11a1 gene expression level in a sample from a subject diagnosed with a papillary lesion of the breast. In this case, putting method (B) into practice comprises determining the col11a1 gene expression level in a sample from said subject diagnosed with a papillary lesion of the breast and comparing said col11a1 gene expression level in said sample with the col11a1 gene expression level in a control sample, and finally correlating the result obtained after said comparison with the prognosis of clinical progression of said papillary lesion and/or with the prediction of risk of recurrence of the papillary lesion of the breast.

Gene expression level can be quantified by means of quantifying the level of the mRNA encoding said gene (col11a1) or, alternatively, the level of complementary DNA (cDNA) to said mRNA. In this case, method (B) comprises performing an extraction step for obtaining total RNA, which can be performed by means of conventional techniques (Chomczynski et al., Anal. Biochem., 1987, 162:156; Chomczynski P., Biotechniques, 1993, 15:532; Molina, M.A., et al., Cancer Res., 1999, 59: 4356-4362).

Virtually any conventional method can be used in the framework of the present invention for quantifying the mRNA level of the col11a1 gene or its corresponding cDNA level. By way of non-limiting illustration, the mRNA level of the col11a1 gene can be quantified by means of using methods comprising mRNA amplification and quantification of said mRNA amplification product, such as electrophoresis and staining, or alternatively by means of Southern blot and the use of suitable probes, Northern blot and the use of specific probes of the mRNA of the col11a1 gene or the corresponding cDNA, mapping with S1 nuclease, RT-LCR, hybridization, microarrays, etc. In a particular embodiment, amplification and quantification of the mRNA corresponding to the col11a1 gene are performed at the same time by means of real time quantitative RT-PCR (Q-PCR). Similarly, the level of the cDNA corresponding to said mRNA of the col11a1 gene can also be quantified by means of using conventional techniques; in this case, the method of the invention includes a synthesis step for synthesizing the corresponding cDNA by means of reverse transcription (RT) of the corresponding mRNA followed by amplification and quantification of said cDNA amplification product. In a particular embodiment, amplification is carried out in a qualitative or quantitative manner by means of a polymerase chain reaction, for example, PCR or RT-PCR, using oligonucleotide primers specifically amplifying a region of the col11a1 gene; in a particular embodiment, said RT-PCR is carried out in the presence of the oligonucleotide primer pair the nucleotide sequences of which are shown in SEQ ID NO: 1 and SEQ ID NO: 2.

According to this particular embodiment of method (B), the mRNA level of the col11a1 gene or the corresponding cDNA level determined in the sample from the subject under study is compared with the mRNA level of the col11a1 gene or the corresponding cDNA level determined in the control sample (reference value). In a particular embodiment, the control sample is a sample from subjects without any medical history of breast tumors or of papillary lesions of the breast (e.g., intraductal papilloma of the breast) without the risk of recurrence in and/or of progression into carcinoma. Additionally, a positive control, for example a cell line expressing the col11a1 gene, and/or a negative control, for example a cell line not expressing the col11a1 gene, can be used if desired.

Additionally, according to this particular embodiment of method (B), the result obtained after comparing the mRNA level of the col11a1 gene or the corresponding cDNA level determined in the sample from the subject under study with the mRNA level of the col11a1 gene or the corresponding cDNA level determined in the control sample (reference value) is correlated with the prognosis of clinical progression of said papillary lesion or with the prediction of risk of recurrence of said papillary lesion of the breast. In a particular embodiment, said papillary lesion of the breast is an intraductal papilloma of the breast. This correlation can indicate that:
- the prognosis of clinical progression of said papillary lesion of the breast is recurrence in carcinoma of the breast when the mRNA level of the col11a1 gene or the corresponding cDNA level determined in the sample from the subject diagnosed with a papillary lesion of the breast is greater than the mRNA level of the col11a1 gene or the corresponding cDNA level determined in the control sample, or that
- the prognosis of clinical progression of said papillary lesion of the breast is no recurrence in carcinoma of the breast when the mRNA level of the col11a1 gene or the corresponding cDNA level determined in the sample from the subject diagnosed with a papillary lesion of the breast is equal to or less than the mRNA level of the col11a1 gene or the corresponding cDNA level determined in the control sample.

This correlation can alternatively indicate that:
- the risk of recurrence of the papillary lesion of the breast is high when the mRNA level of the col11a1 gene or the corresponding cDNA level determined in the sample from the subject diagnosed with a papillary lesion of the breast is greater than the mRNA level of the col11a1 gene or the corresponding cDNA level determined in the control sample, or that
- the risk of recurrence of the papillary lesion of the breast is low when the mRNA level of the col11a1 gene or the corresponding cDNA level determined in the sample from the subject diagnosed with a papillary lesion of the breast is equal to or less than the mRNA level of the col11a1 gene or the corresponding cDNA level determined in the control sample.

This information can be used efficiently by specialists for selecting the most suitable treatment to be administered to the subject diagnosed with a papillary lesion of the breast according to the risk of recurrence of said papillary lesion of the breast.

Given the lack of reliable markers, there is enormous debate today as to the treatment of papillary lesions of the breast, and particularly of intraductal papilloma of the breast (a benign papillary lesion of the breast with a fairly high probability of recurrence in a malignant pathology (33%) when the initial lesion presents atypias), so some specialists advocate performing a complete removal of the lesion in all cases, whereas other specialists advocate the complete removal of the lesion when atypias or multiple papillomas present.

Now, by means of the information provided by the first method of the invention [method (A) and method (B)], specialists may select the most suitable treatment to be administered to the subject diagnosed with a papillary lesion of the breast according to the risk of recurrence of said papillary lesion of the breast, thus optimizing the therapeutic care to be applied to the subject and avoiding the drawbacks associated with applying unsuitable treatment.

In a particular embodiment, the papillary lesion of the breast that the subject under study suffers is an intraductal papilloma of the breast.

In another particular embodiment, the recurrence of the papillary lesion of the breast is a recurrence in a carcinoma of the breast or breast tumor.

In another particular embodiment, the recurrence of the papillary lesion of the breast is a recurrence in ductal adenocarcinoma, ductal carcinoma *in situ,* invasive ductal carcinoma, lobular carcinoma, lobular carcinoma *in situ,* invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer.

### Selection methods

There are different treatments that can be applied to a subject diagnosed with a papillary lesion of the breast although there are discrepancies as to the specific treatment to be applied. By way of illustration, some specialists advocate treating papillary lesions of the breast by means of surgical removal of the breast; other specialists advocate treating said lesions by means of surgical removal of the papillary lesion of the breast by means of conservative surgery, for example, but without being limited to resection by mammotome or resection by cores. Sometimes these treatments are accompanied by pharmacological treatment and/or subsequent radiation therapy treatment and by follow-up of the progression of the lesions and/or of the treatment applied with a specialist.

It has now been found that since it is possible to correlate detection of the presence of proCOL11A1 protein and/or quantification of the proCOL11A1 protein expression level in a sample from a subject diagnosed with a papillary lesion of the breast with the prognosis of its clinical progression and/or with the prediction of risk of recurrence of said lesion, the specialist can choose the most suitable treatment to be applied to said subject according to the risk of recurrence of said papillary lesion of the breast. Therapeutic care for the subject is thus optimized, and drawbacks associated with applying unsuitable treatment are avoided, for example, unnecessary surgical interventions are avoided with the subsequent economic repercussion this entails.

Therefore, in another aspect the invention relates to a method for selecting a subject diagnosed with a papillary lesion of the breast for treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence, hereinafter "second method of the invention", which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said subject is selected for said treatment:
- if the presence of proCOL11A1 protein is detected in said sample from the subject diagnosed with a papillary lesion of the breast; or
- if the proCOL11A1 protein expression level in said sample from the subject diagnosed with a papillary lesion of the breast is greater than said proCOL11A1 protein expression level in the control sample.

Therefore, according to the second method of the invention, the presence of proCOL11A1 protein is detected and/or said proCOL11A1 protein expression level is determined in a sample from the subject diagnosed with a papillary lesion of the breast that is being evaluated, and the obtained result is correlated with the possibility of selecting said subject to receive treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence.

The characteristics of the sample, the subject to be treated and the proCOL11A1 protein have already been mentioned above in relation to the first method of the invention, just like the methods for detecting and/or quantifying said protein, and are herein incorporated by reference.

In a particular embodiment, treatment comprising surgical removal of said papillary lesion of the breast by means of a method that allows assuring complete removal of the lesion comprises performing a partial or total mastectomy, for example, includes treatment ranging from conservative surgery of the breast to Mérola-Patey's modified radical mastectomy, as the case may be. Specialists in the field can apply the preventive pharmacological treatment that they consider most suited to the case to prevent recurrence. Specialists may also perform comprehensive follow-up of the progression of the lesion for the purpose of early detection of the possible recurrence, for which purpose they will establish guidelines which they consider to be most pertinent, as the case may be.

Once the presence and/or the amount of proCOL11A1 protein in the sample from the subject diagnosed with a papillary lesion of the breast under study is analyzed, it is possible to evaluate the possibility of selecting said subject to receive treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence. The possibility of selecting a subject to receive said treatment is established based on detection of the presence of proCOL11A1 protein or on said protein expression level in a sample from the subject diagnosed with a papillary lesion of the breast, such that if the presence of proCOL11A1 protein is detected in said sample from the subject diagnosed with a papillary lesion of the breast, or if the proCOL11A1 protein expression level in said sample from the subject diagnosed with a papillary lesion of the breast is greater than said proCOL11A1 protein expression level in the control sample, then said subject is selected for said treatment. Otherwise, the subject will not be selected for that treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence. In a particular embodiment, said papillary lesion of the breast is an intraductal papilloma of the breast.

The second method of the invention therefore allows selecting a subject diagnosed with a papillary lesion of the breast in need of treatment, to receive treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence. Therefore, if the presence of proCOL11A1 protein is detected in the analyzed sample from the subject, or if the proCOL11A1 protein expression level in said sample is greater than said proCOL11A1 protein expression level in a control sample, then said subject is, *a priori,* a suitable candidate to receive treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence.

This second method of the invention is a valuable tool for the treatment of papillary lesions of the breast because in view of the results provided by evaluating the risk of recurrence of a papillary lesion of the breast by means of a method such as the one provided by this invention, specialists may select (or disregard) the subject that is being evaluated to receive treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence according to detection of the presence of proCOL11A1 protein, or to the proCOL11A1 protein expression level in the sample from said subject. Therefore, the methods and means provided by the present invention can help specialists select the most suitable treatment to be administered to a subject with papillary lesion of the breast and avoid the adverse effects associated with the administration of unnecessary treatment in the case of subjects without the risk of recurrence or progression of a papillary lesion into carcinoma of the breast.

Alternatively, in another aspect the invention provides a method for selecting a subject diagnosed with a papillary lesion of the breast for treatment selected from treatment comprising the removal of said papillary lesion of the breast by conservative surgery and treatment comprising non-removal of said papillary lesion of the breast, hereinafter "third method of the invention", which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said subject is selected for said treatment:
- if the presence of proCOL11A1 protein is not detected in said sample from the subject diagnosed with a papillary lesion of the breast; or
- if the proCOL11A1 protein expression level in said sample from the subject diagnosed with a papillary lesion of the breast is equal to or less than said proCOL11A1 protein expression level in the control sample.

According to the third method of the invention, proCOL11A1 protein is detected and/or said proCOL11A1 protein expression level is determined in a sample from the subject diagnosed with a papillary lesion of the breast that is being evaluated, and the obtained result is correlated with the possibility of selecting said subject to receive treatment selected from (i) treatment comprising the removal of said papillary lesion of the breast by conservative surgery, and (ii) treatment comprising non-removal of said papillary lesion of the breast.

In a particular embodiment, treatment comprising surgical removal of the papillary lesion of the breast by means of conservative surgery is carried out by means of mammotome or cores.

The characteristics of the sample, the subject to be treated and the proCOL11A1 protein have already been mentioned above in relation to the first method of the invention, just like methods for detecting and/or quantifying said protein, and are herein incorporated by reference.

Once the presence and/or the amount of proCOL11A1 protein in the sample from the subject diagnosed with a papillary lesion of the breast under study is analyzed, it is possible to evaluate the possibility of selecting said subject to receive treatment selected from (i) treatment comprising the removal of said papillary lesion of the breast by conservative surgery, and (ii) treatment comprising non-removal of said papillary lesion of the breast. The possibility of selecting a subject diagnosed with a papillary lesion of the breast to receive said treatment is established based on detection of the presence of proCOL11A1 protein or on said protein expression level in a sample from the subject diagnosed with a papillary lesion of the breast, such that if the presence of proCOL11A1 protein is not detected in said sample from the subject diagnosed with a papillary lesion of the breast, or if the proCOL11A1 protein expression level in said sample from the subject diagnosed with a papillary lesion of the breast is equal to or less than said proCOL11A1 protein expression level in the control sample, then said subject is selected for said treatment. Otherwise, the subject will not be selected for that treatment selected from (i) treatment comprising the removal of said papillary lesion of the breast by conservative surgery, and (ii) treatment comprising non-removal of said papillary lesion of the breast. In a particular embodiment, said papillary lesion of the breast is an intraductal papilloma of the breast.

In a particular embodiment, specialists select the subject under study for treatment comprising the removal of the papillary lesion of the breast by conservative surgery. In a specific embodiment, treatment comprising the removal of the papillary lesion of the breast by conservative surgery comprises resection by mammotome, resection of said papillary lesion of the breast by cores, or en bloc resection comprising the duct involved.

In another particular embodiment, specialists select the subject under study for treatment not comprising the removal of the papillary lesion of the breast by conservative surgery.

The third method of the invention therefore allows selecting a subject diagnosed with a papillary lesion of the breast in need of treatment to receive treatment selected from (i) treatment comprising the removal of said papillary lesion of the breast by conservative surgery, and (ii) treatment comprising non-removal of said papillary lesion of the breast. Therefore, if the presence of proCOL11A1 protein is not detected in the analyzed sample from the subject, or if the proCOL11A1 protein expression level in said sample is equal to or less than said proCOL11A1 protein expression level in a control sample, then said subject is, *a priori,* a suitable candidate to receive said treatment mentioned above.

This third method of the invention is a valuable tool for the treatment of papillary lesions of the breast because in view of the results provided by evaluating the risk of recurrence of a papillary lesion of the breast by means of a method such as the one provided by this invention, specialists may select (or disregard) the subject that is being evaluated to receive treatment selected from (i) treatment comprising the removal of said papillary lesion of the breast by conservative surgery, and (ii) treatment comprising non-removal of said papillary lesion of the breast, according to detection of the presence of proCOL11A1 protein or to the proCOL11A1 protein expression level in the sample from said subject. Therefore, the methods and means provided by the present invention can help specialists select the most suitable treatment to be administered to a subject with papillary lesion of the breast and avoid the adverse effects associated with the administration of ineffective treatment.

In another aspect, the invention relates to a method for selecting treatment for a subject diagnosed with a papillary lesion of the breast, hereinafter "fourth method of the invention", which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said treatment is selected from pharmacological treatment, treatment comprising surgical removal of said papillary lesion of the breast, and a combination thereof,
wherein said treatment is selected:
- if the presence of proCOL11A1 protein is detected in said sample from the subject diagnosed with a papillary lesion of the breast; or
- if the proCOL11A1 protein expression level in said sample from the subject diagnosed with a papillary lesion of the breast is greater than said proCOL11A1 protein expression level in the control sample.

According to the fourth method of the invention, proCOL11A1 protein is detected and/or said proCOL11A1 protein expression level is determined in a sample from the subject diagnosed with a papillary lesion of the breast that is being evaluated, and the obtained result is correlated with the possibility of selecting treatment for a subject diagnosed with a papillary lesion of the breast, wherein said treatment is selected from pharmacological treatment, treatment comprising surgical removal of said papillary lesion of the breast, and a combination thereof.

The characteristics of the sample, the subject to be treated and the proCOL11A1 protein have already been mentioned above in relation to the first method of the invention, just like the methods for detecting and/or quantifying said protein, and are herein incorporated by reference.

Once the presence and/or the amount of proCOL11A1 protein in the sample from the subject diagnosed with a papillary lesion of the breast under study is analyzed, it is possible to evaluate the possibility of selecting treatment for a subject diagnosed with a papillary lesion of the breast, wherein said treatment is selected from pharmacological treatment, treatment comprising surgical removal of said papillary lesion of the breast, and a combination thereof. The possibility of selecting said treatment for a subject diagnosed with a papillary lesion of the breast is established based on detection of the presence of proCOL11A1 protein or on said protein expression level in a sample from the subject diagnosed with a papillary lesion of the breast, such that if the presence of proCOL11A1 protein is detected in said sample from the subject diagnosed with a papillary lesion of the breast, or if the proCOL11A1 protein expression level in said sample from the subject diagnosed with a papillary lesion of the breast is greater than said proCOL11A1 protein expression level in the control sample, then treatment selected from pharmacological treatment, treatment comprising surgical removal of said papillary lesion of the breast, and a combination thereof, is selected to be applied to said subject. Otherwise, that treatment will not be selected for that subject with papillary lesion of the breast. In a particular embodiment, said papillary lesion of the breast is an intraductal papilloma of the breast.

In a particular embodiment, specialists select pharmacological treatment to be applied to a subject diagnosed with a papillary lesion of the breast. Specialists in the field will choose the pharmacological treatment that they consider most suited to the case to be treated; illustrative, non-limiting examples of drugs that can be used in said pharmacological treatment include docetaxel, doxorubicin, 5-fluorouracil, methotrexate, cyclophosphamide, etc. Therefore in a particular embodiment, pharmacological treatment comprises administration of a drug suitable for the treatment of a papillary lesion of the breast, such as docetaxel, doxorubicin, 5-fluorouracil, methotrexate, cyclophosphamide, for example, or generally any drug that the specialists consider suitable.

In another particular embodiment, specialists select treatment comprising surgical removal of said papillary lesion of the breast to be applied to a subject diagnosed with a papillary lesion of the breast. Surgical removal of said papillary lesion of the breast can be performed by means of any of the techniques mentioned above in this description or any other technique that the specialists in the field consider suitable.

In another particular embodiment, specialists select treatment comprising surgical removal of said papillary lesion of the breast to be applied to a subject diagnosed with a papillary lesion of the breast and pharmacological treatment.

In another particular embodiment, the fourth method of the invention further comprises subsequent radiation therapy treatment, if desired, that can include but is not limited to external radiation therapy, locoregional radiation therapy or internal radiation therapy/brachytherapy, and/or follow-up of the progression of the disease or of the treatment applied during a suitable time period according to the specialist.

The fourth method of the invention therefore allows selecting treatment selected from pharmacological treatment, treatment comprising surgical removal of said papillary lesion of the breast, and a combination thereof, for a subject diagnosed with a papillary lesion of the breast. Therefore, if the presence of proCOL11A1 protein is detected in the analyzed sample from the subject, or if the proCOL11A1 protein expression level in said sample is greater than said proCOL11A1 protein expression level in a control sample, then said subject is, *a priori,* a suitable candidate to receive said treatment selected from pharmacological treatment, treatment comprising surgical removal of said papillary lesion of the breast, and a combination thereof.

This fourth method of the invention is a valuable tool for the treatment of papillary lesions of the breast because in view of the results provided by evaluating the risk of recurrence of a papillary lesion of the breast by means of a method such as the one provided by this invention, specialists may select (or disregard) the (pharmacological and/or surgical) treatment to be applied to the subject that is being evaluated, according to detection of the presence of proCOL11A1 protein or to the proCOL11A1 protein expression level in the sample from said subject. Therefore, the methods and means provided by the present invention can help specialists select the most suitable treatment to be administered to a subject with papillary lesion of the breast and avoid the adverse effects associated with the administration of ineffective treatment.

### Uses

In another aspect, the invention relates to the use of proCOL11A1 protein as a marker for prognosticating clinical progression or for predicting the risk of recurrence of a papillary lesion of the breast, or for selecting a subject diagnosed with a papillary lesion of the breast, or for selecting treatment for a subject diagnosed with a papillary lesion of the breast.

In a particular embodiment, the papillary lesion of the breast is an intraductal papilloma of the breast.

In another particular embodiment, the recurrence of the papillary lesion of the breast is a recurrence in a carcinoma of the breast or breast tumor. In another particular embodiment, said recurrence is a recurrence in ductal adenocarcinoma, ductal carcinoma *in situ,* invasive ductal carcinoma, lobular carcinoma, lobular carcinoma *in situ,* invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer.

In another aspect, the invention relates to the use of a specific antibody recognizing proCOL11A1 protein, or a fragment thereof recognizing proCOL11A1 protein, as a marker for prognosticating clinical progression or for predicting the risk of recurrence of a papillary lesion of the breast, or for selecting a subject diagnosed with a papillary lesion of the breast, or for selecting treatment for a subject diagnosed with a papillary lesion of the breast.

The characteristics of the specific antibody recognizing proCOL11A1 protein, or a fragment thereof recognizing proCOL11A1 protein, have been mentioned in relation to the first method of the invention, particularly in relation to method (A), and are herein incorporated by reference. The antibody recognizing proCOL11A1 protein is advantageously an antibody specifically recognizing the VAR subdomain in the N-terminal propeptide of proCOL11A1. Illustrative examples of antibodies specifically detecting proCOL11A1 are mentioned in international patent application PCT/ES2012/070616 [WO 2013/021088]. In a particular embodiment, said antibody is monoclonal antibody 1E8.33 (Garcia-Ocaña, et al., Int J Oncol. 2012; 40(5): 1447-54), an antibody specifically recognizing proCOL11A1 protein, without detecting other proteins with a high sequence homology, such as COL5A1 protein.

In a particular embodiment, the papillary lesion of the breast is an intraductal papilloma of the breast.

In another particular embodiment, the recurrence of the papillary lesion of the breast is a recurrence in a carcinoma of the breast or breast tumor. In another particular embodiment, said recurrence is a recurrence in ductal adenocarcinoma, ductal carcinoma *in situ,* invasive ductal carcinoma, lobular carcinoma, lobular carcinoma *in situ,* invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer.

In another aspect, the invention relates to the use of a kit comprising a reagent recognizing proCOL11A1 protein, or a reagent for detection and/or quantification of col11a1 gene expression, for prognosticating clinical progression and/or for predicting the risk of recurrence of a papillary lesion of the breast, or for selecting a subject diagnosed with a papillary lesion of the breast, or for selecting treatment for a subject diagnosed with a papillary lesion of the breast.

In a particular embodiment, said reagent recognizing proCOL11A1 protein is a specific antibody recognizing proCOL11A1 protein or a fragment thereof recognizing proCOL11A1 protein. The characteristics of said specific antibody recognizing proCOL11A1 protein or fragment thereof recognizing proCOL11A1 protein have been mentioned in relation to the first method of the invention, particularly in relation to method (A) and are herein incorporated by reference. The antibody recognizing proCOL11A1 protein is advantageously an antibody specifically recognizing the VAR subdomain in the N-terminal propeptide of proCOL11A1. Illustrative examples of antibodies specifically detecting proCOL11A1 are mentioned in international patent application PCT/ES2012/070616 [WO 2013/021088]. In a particular embodiment, said antibody is monoclonal antibody 1E8.33 (Garcia-Ocaña, et al., Int J Oncol. 2012; 40(5): 1447-54), an antibody specifically recognizing proCOL11A1 protein, without detecting other proteins with a high sequence homology, such as COL5A1 protein.

In another particular embodiment, said reagent for detection and/or quantification of col11a1 gene expression comprises an oligonucleotide primer pair specifically amplifying a region of the col11a1 gene and/or a nucleotide probe recognizing a region of the col11a1 gene; in a particular embodiment, said oligonucleotide primer pair comprises oligonucleotides the nucleotide sequences of which are shown in SEQ ID NO: 1 and SEQ ID NO: 2.

In a particular embodiment, the recurrence of the papillary lesion of the breast is a recurrence in a carcinoma of the breast or breast tumor. In another particular embodiment, said recurrence is a recurrence in ductal adenocarcinoma, ductal carcinoma *in situ*, invasive ductal carcinoma, lobular carcinoma, lobular carcinoma *in situ*, invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer.

In another particular embodiment, the papillary lesion of the breast is an intraductal papilloma of the breast.

In view of the foregoing, the person skilled in the art will comprehend that the invention furthermore allows classifying papillary lesions of the breast, particularly intraductal papillomas of the breast, according to their tendency or their risk to recur in and/or progress into carcinomas (becoming malignant), which is useful for deciding on the most suitable treatment and follow-up regimen for each subject diagnosed with a papillary lesion of the breast by means of detection of the presence of proCOL11A1 protein or quantification of said proCOL11A1 protein expression level in a sample from said subject. Therefore, the invention provides methods and products for the highly sensitive and specific *in vitro* detection of those papillary lesions of the breast, particularly those intraductal papillomas of the breast, with the risk of progressing into aggressive lesions and, therefore, susceptible to resection (i.e., being surgically removed) at the time of diagnosis and subjected to a subsequent and more comprehensive follow-up, and distinguishing same from those papillary lesions of the breast, particularly those intraductal papillomas of the breast that are benign and do not require an aggressive therapeutic approach.

The following example illustrates the invention and must not be considered in sense that limits said invention.

### EXAMPLE 1

### Differential expression of proCOL11A1 in intraductal papillomas of the breast and correlation with biological aggressiveness

### 1.1 Materials and Methods

### Immunohistochemistry

Sections of the core needle biopsies (CNB), fixed in formol and included in paraffin, from patients with at least 5 years of follow-up after extraction of the histological section were cut with a microtome. The 3-4 µm sections were dried overnight at 54-56°C in an oven. Pre-treatment with CC2 [Cell Conditioning 2] buffer [Ventana Medical Systems, Inc.; catalog no. 950-123] was applied at 98°C for 32 minutes. After titration to adjust the suitable antibody concentration, between 65, 26 and 13 µg/ml, the preparations were incubated with monoclonal antibody 1E8.33 specific for proCOL11A1 at a concentration of 26 µg/ml in Antibody diluent (Ventana-Roche, Tucson, Arizona) for 32 minutes at room temperature. The "Optiview" (Ventana) detection system was used and developed with diaminobenzidine (DAB) (Ventana).

Sixty-one cases of intraductal papilloma were analyzed with monoclonal antibody 1E8.33 specific for proCOL11A1. The immunostaining was evaluated with a double-blind assay conducted by 2 different observers. It was considered positive when at least one cell with a fibroblastic morphology has an immunoprecipitate.

### 1.2 Statistical data analysis

Fisher's exact test was used to calculate the distribution of frequencies. p<0.05 was assumed to be significant.

### 1.3 Results

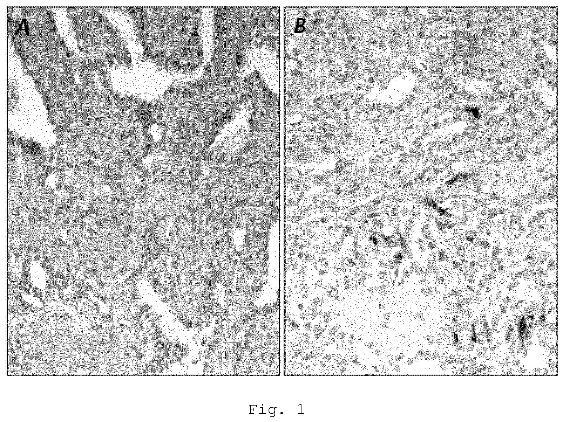

ProCOL11A1 protein expression was studied in histological sections of intraductal papilloma biopsy cores from patients with at least 5 years of follow-up after extraction of the histological section analyzed with specific antibodies of the proCOL11A1 protein (1E8.33). The objective of these experiments was to determine if the presence of proCOL11A1 immunolabeling could be associated with a malignant recurrence. Twenty-six of the 61 cases analyzed were positive for the immunolabeling with the specific antibody of the proCOL11A1 protein, i.e., they presented proCOL11A1 protein expression, and 35 were negative (Figure 1A).

Of the 26 positive cases, 10 patients presented malignant recurrence in the form of infiltrating carcinoma of the breast, after at least 5 years of follow-up (Figure 1B), 3 patients presented recurrence in the form of benign papilloma or lesion, and 13 patients presented no recurrence as of the time of the immunohistochemical analysis of the tissues.

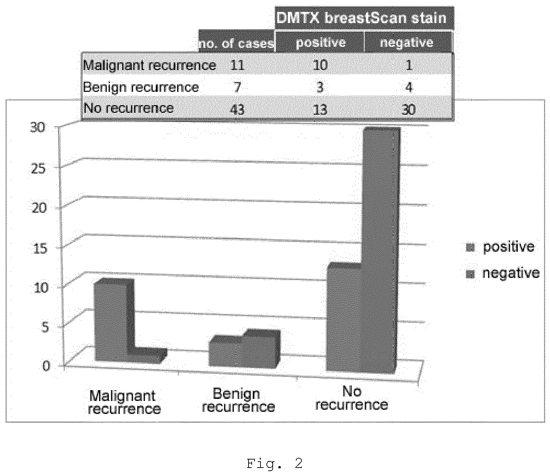

Out of the 35 negative cases, without proCOL11A1 expression, only one patient presented recurrence in the form of infiltrating carcinoma, and 4 patients presented benign recurrence. The rest (30 patients) did not present any type of recurrence in the 5 years of follow-up that were studied (Figure 2).

According to Fischer's test, the frequency of positive staining for proCOL11A1 showed statistically significant differences (p<0.005) between the group of patients with malignant recurrences and the group of patients with benign recurrences, and between the group of patients with malignant recurrences and the group of patients without recurrence.

Furthermore, sensitivity for predicting recurrences (72%), sensitivity for predicting malignant recurrences (91%), specificity for detecting recurrences (70%) and specificity for detecting malignant recurrences (68%), as well as the positive predictive values (PPVs) and negative predictive values (NPVs) for detecting recurrences (69% PPVs and 88% NPVs) and malignant recurrences (61% PPV and 97% NPV), were calculated. These results demonstrate that this marker (proCOL11A1) is highly sensitive for predicting recurrences, being even greater for predicting malignant recurrences.

### 1.4 Conclusions

Immunostaining with monoclonal antibody 1E8.33, which is specific for proCOL11A1, is highly sensitive and specific in the detection of patients with intraductal papilloma of the breast that are going to develop a recurrence or a malignant recurrence.

## Claims

1. An *in vitro* method for prognosticating clinical progression and/or predicting the risk of recurrence of a papillary lesion of the breast, selected from method (A) and method (B), wherein
A) method (A) comprises
- detecting the presence of proCOL11A1 protein in a sample from a subject diagnosed with a papillary lesion of the breast;
wherein
- detection of the presence of proCOL11A1 protein in said sample from a subject diagnosed with a papillary lesion of the breast is indicative that the risk of recurrence is high, or
- non-detection of the presence of proCOL11A1 protein in said sample from a subject diagnosed with a papillary lesion of the breast is indicative that the risk of recurrence is low, and
B) method (B) comprises
- comparing the proCOL11A1 protein expression level in said sample with said proCOL11A1 protein expression level in a control sample;
wherein
- a proCOL11A1 protein expression level in said sample from a subject diagnosed with a papillary lesion of the breast greater than said proCOL11A1 protein expression level in a control sample is indicative that the risk of recurrence is high, or
- a proCOL11A1 protein expression level in said sample from a subject diagnosed with a papillary lesion of the breast equal to or less than said proCOL11A1 protein expression level in a control sample is indicative that the risk of recurrence is low.

2. The method according to claim 1, wherein said sample is a breast tissue sample or a biological fluid sample.

3. The method according to any of claims 1 or 2, which further comprises obtaining from said sample a protein extract or an extract comprising total RNA.

4. The method according to any of claims 1 to 3, wherein the detection of proCOL11A1 protein comprises contacting the breast tissue, or the protein extract of the sample, with an antibody specific for proCOL11A1 protein, under conditions that allow forming an antibody-proCOL11A1 protein complex.

5. The method according to claim 4, wherein said antibody specific for proCOL11A1 protein is an antibody recognizing an epitope located in the VAR domain of the N-terminal end of proCOL11A1 protein.

6. The method according to claim 4 or 5, wherein said antibody specific for proCOL11A1 protein is monoclonal antibody 1E8.33.

7. The method according to any of claims 4 to 6, wherein the detection and/or quantification of the antibody-proCOL11A1 protein complex formed is carried out by means of a technique selected from the group consisting of Western-blot, ELISA, RIA, competitive EIA, DAS-ELISA, immunocytochemical and immunohistochemical techniques, multiplex detection techniques based on the use of protein microspheres, biochips or microarrays including specific antibodies, or assays based on colloidal precipitation.

8. The method according to any of the preceding claims, wherein said recurrence is a recurrence in a carcinoma of the breast or breast tumor.

9. The method according to any of the preceding claims, wherein said recurrence is a recurrence in ductal adenocarcinoma, ductal carcinoma *in situ*, invasive ductal carcinoma, lobular carcinoma, lobular carcinoma *in situ*, invasive lobular carcinoma, tubular carcinoma, medullary carcinoma, mucinous or colloid carcinoma, papillary carcinoma, inflammatory carcinoma, cribriform carcinoma, Paget's disease of the breast, invasive breast cancer or metastatic breast cancer.

10. The method according to any of the preceding claims, wherein said papillary lesion of the breast is an intraductal papilloma of the breast.

11. A method for selecting a subject diagnosed with a papillary lesion of the breast for treatment comprising surgical removal of said papillary lesion of the breast by means of a method that allows assuring complete removal of the lesion and preventive pharmacological treatment to prevent recurrence, as well as comprehensive follow-up of the progression of the lesion, which allows early detection of the possible recurrence, which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said subject is selected for said treatment:
- if the presence of proCOL11A1 protein is detected in said sample from the subject diagnosed with a papillary lesion of the breast; or
- if the proCOL11A1 protein expression level in said sample from the subject diagnosed with a papillary lesion of the breast is greater than said proCOL11A1 protein expression level in the control sample.

12. The method according to claim 11, wherein said treatment comprising surgical removal of said papillary lesion of the breast by means of a method that allows assuring complete removal of the lesion comprises performing a partial or total mastectomy.

13. A method for selecting a subject diagnosed with a papillary lesion of the breast for treatment selected from treatment comprising the removal of said papillary lesion of the breast by conservative surgery and treatment comprising non-removal of said papillary lesion of the breast, which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said subject is selected for said treatment:
- if the presence of proCOL11A1 protein is not detected in said sample from the subject diagnosed with a papillary lesion of the breast; or
- if the proCOL11A1 protein expression level in said sample from the subject diagnosed with a papillary lesion of the breast is equal to or less than said proCOL11A1 protein expression level in the control sample.

14. The method according to claim 13, wherein said treatment comprising the removal of the papillary lesion of the breast by conservative surgery comprises resection by microtome, resection of said papillary lesion of the breast by cores, or en bloc resection comprising the duct involved.

15. A method for selecting treatment for a subject diagnosed with a papillary lesion of the breast, which comprises
- detecting the presence of proCOL11A1 protein in a sample from a subject diagnosed with a papillary lesion of the breast; or
- comparing the proCOL11A1 protein expression level in said sample with said proCOL11A1 protein expression level in a control sample;
wherein said treatment is selected from pharmacological treatment, treatment comprising surgical removal of said papillary lesion of the breast, and a combination thereof,
wherein said treatment is selected:
- if the presence of proCOL11A1 protein is detected in said sample from the subject diagnosed with a papillary lesion of the breast; or
- if the proCOL11A1 protein expression level in said sample from the subject diagnosed with a papillary lesion of the breast is greater than said proCOL11A1 protein expression level in the control sample.

16. The method according to claim 15, wherein said pharmacological treatment comprises the administration of a drug selected from the group consisting of docetaxel, doxorubicin, 5-fluorouracil, methotrexate, cyclophosphamide, or their combinations.

17. The method according to claim 15 or 16, wherein said treatment further comprises subsequent radiation therapy treatment.

## Patentansprüche

1. Ein *in vitro*-Verfahren zur Prognose des klinischen Verlaufs und/oder Vorhersage des Risikos eines Wiederauftretens einer papillären Mammaläsion, ausgewählt aus Verfahren (A) und Verfahren (B), wobei
A) Verfahren (A) umfasst
- Nachweisen des Vorhandenseins des proCOL11A1-Proteins in einer Probe von einer Person, bei der eine papilläre Mammaläsion diagnostiziert wurde; wobei
- der Nachweis des Vorhandenseins des proCOL11A1-Proteins in der Probe von einer Person, bei der eine papilläre Mammaläsion diagnostiziert wurde, ein Anzeichen ist, dass das Risiko eines Wiederauftretens hoch ist, oder
- das Nichterkennen des Vorhandenseins des proCOL11A1-Proteins in der Probe von einer Person, bei der eine papilläre Mammaläsion diagnostiziert wurde, ein Anzeichen ist, dass das Risiko eines Wiederauftretens gering ist, und
B) Verfahren (B) umfasst
- Vergleichen des proCOL11A1-Protein-Expressionsniveaus in der Probe mit dem proCOL11A1-Protein-Expressionsniveau in einer Kontrollprobe;
wobei
- ein proCOL11A1-Protein-Expressionsniveau in der Probe von einer Person, bei der eine papilläre Mammaläsion diagnostiziert wurde, das größer ist als das proCOL11A1-Protein-Expressionsniveau in einer Kontrollprobe, ein Anzeichen ist, dass das Risiko eines Wiederauftretens hoch ist, oder
- ein proCOL11A1-Protein-Expressionsniveau in der Probe von einer Person, bei der eine papilläre Mammaläsion diagnostiziert wurde, das gleich oder geringer ist als das proCOL11A1-Protein-Expressionsniveau in einer Kontrollprobe, ein Anzeichen ist, dass das Risiko eines Wiederauftretens gering ist.

2. Das Verfahren gemäß Anspruch 1, wobei die Probe eine Brustgewebeprobe oder eine Probe einer biologischen Flüssigkeit ist.

3. Das Verfahren gemäß irgendeinem der Ansprüche 1 oder 2, das weiterhin das Erhalten eines Proteinextraktes oder eines Extraktes enthaltend die Gesamt-RNA aus der Probe umfasst.

4. Das Verfahren gemäß irgendeinem der Ansprüche 1 bis 3, wobei der Nachweis des proCOL11A1-Proteins das In-Kontakt-Bringen des Brustgewebes oder des Proteinextraktes der Probe mit einem Antikörper umfasst, der spezifisch für das proCOL11A1-Protein ist, unter Bedingungen, die die Bildung eines Antikörper-proCOL11A1-Protein-Komplexes ermöglichen.

5. Das Verfahren gemäß Anspruch 4, wobei der für das proCOL11A1-Protein spezifische Antikörper ein Antikörper ist, der ein Epitop erkennt, das in der VAR-Domäne des N-terminalen Endes des proCOL11A1-Proteins lokalisiert ist.

6. Das Verfahren gemäß Anspruch 4 oder 5, wobei der für das proCOL11A1-Protein spezifische Antikörper der monoklonale Antikörper 1E8.33 ist.

7. Das Verfahren gemäß irgendeinem der Ansprüche 4 bis 6, wobei der Nachweis und/oder die Quantifizierung des gebildeten Antikörper-proCOL11A1-Protein-Komplexes mittels einer Technik durchgeführt wird, die ausgewählt ist aus der Gruppe bestehend aus Western-Blot, ELISA, RIA, kompetetive EIA, DAS-ELISA, immunzytochemische und immunhistochemische Techniken, Multiplex-Detektionstechniken basierend auf der Verwendung von Protein-Mikrokügelchen, Biochips oder Mikroarrays einschließlich spezifischer Antikörper oder auf kolloidaler Fällung basierende Assays.

8. Das Verfahren gemäß irgendeinem der vorhergehenden Ansprüche, wobei das Wiederauftreten ein Rezidiv in einem Karzinom der Brust oder des Brusttumors ist.

9. Das Verfahren gemäß irgendeinem der vorhergehenden Ansprüche, wobei das Wiederauftreten ein Rezidiv in einem duktalen Adenokarzinom, duktalen Karzinom *in situ,* invasiven duktalen Karzinom, lobulären Karzinom, lobulären Karzinom *in situ,* invasiven lobulären Karzinom, tubulären Karzinom, medullären Karzinom, muzinösen oder Kolloidkarzinom, Papillarkarzinom, entzündlichen Karzinom, kribriformen (siebartigen) Karzinom, einer Paget-Erkrankung der Brust, invasiven Brustkrebs oder metastatischen Brustkrebs ist.

10. Das Verfahren gemäß irgendeinem der vorhergehenden Ansprüche, wobei die papilläre Mammaläsion ein intraduktales Papillom der Brust ist.

11. Ein Verfahren zur Auswahl einer Person, bei der eine papilläre Mammaläsion diagnostiziert wurde, zur Behandlung umfassend die chirurgische Entfernung der papillären Mammaläsion mittels eines Verfahrens, das eine vollständige Entfernung der Läsion gewährleistet, und präventive pharmakologische Behandlung, zur Vermeidung des Wiederauftretens, sowohl als auch eine umfassende Nachverfolgung des Verlaufs der Läsion, die eine Früherkennung eines möglichen Wiederauftretens erlaubt, umfassend
- Nachweisen des Vorhandenseins des proCOL11A1-Proteins in einer Probe von einer Person, bei der eine papilläre Mammaläsion diagnostiziert wurde; oder
- Vergleichen des proCOL11A1-Protein-Expressionsniveaus in der Probe mit dem proCOL11A1-Protein-Expressionsniveau in einer Kontrollprobe;
wobei die Person für die Behandlung ausgewählt wird:
- wenn das Vorhandensein des proCOL11A1-Proteins in der Probe von der Person, bei der eine papilläre Mammaläsion diagnostiziert wurde, nachgewiesen wird; oder
- wenn das proCOL11A1-Protein-Expressionsniveau in der Probe von der Person, bei der eine papilläre Mammaläsion diagnostiziert wurde, größer ist als das proCOL11A1-Proteinexpressionsniveau in der Kontrollprobe.

12. Das Verfahren gemäß Anspruch 11, wobei die Behandlung, die die chirurgische Entfernung der papillären Mammaläsion mittels eines Verfahrens beinhaltet, das die vollständige Entfernung der Läsion gewährleistet, die Durchführung einer teilweisen oder vollständigen Mastektomie umfasst.

13. Ein Verfahren zur Auswahl einer Person, bei der eine papilläre Mammaläsion diagnostiziert wurde, zur Behandlung ausgewählt aus einer Behandlung umfassend das Entfernen der papillären Mammaläsion durch konservative Chirurgie und einer Behandlung, die die Nicht-Entfernung der papillären Mammaläsion beinhaltet, umfassend
- Nachweisen des Vorhandenseins des proCOL11A1-Proteins in einer Probe von einer Person, bei der eine papilläre Mammaläsion diagnostiziert wurde; oder
- Vergleichen des proCOL11A1-Protein-Expressionsniveaus in der Probe mit dem proCOL11A1-Protein-Expressionsniveau in einer Kontrollprobe;
wobei die Person für die Behandlung ausgewählt wird:
- wenn das Vorhandensein des proCOL11A1-Proteins in der Probe von der Person, bei der eine papilläre Mammaläsion diagnostiziert wurde, nicht nachgewiesen wird; oder
- wenn das proCOL11A1-Protein-Expressionsniveau in der Probe von der Person, bei der eine papilläre Mammaläsion diagnostiziert wurde, gleich oder geringer ist als das proCOL11A1-Proteinexpressionsniveau in der Kontrollprobe.

14. Das Verfahren gemäß Anspruch 13, wobei die Behandlung, die die Entfernung der papillären Mammaläsion durch konservative Chirurgie beinhaltet, eine Resektion durch Mikrotom, eine Resektion der papillären Mammaläsion mittels Inzision oder eine En-bloc-Resektion einschließlich des beteiligten Kanals umfasst.

15. Ein Verfahren zur Auswahl einer Behandlung für eine Person, bei der eine papilläre Mammaläsion diagnostiziert wurde, umfassend
- Nachweisen des Vorhandenseins des proCOL11A1-Proteins in einer Probe von einer Person, bei der eine papilläre Mammaläsion diagnostiziert wurde; oder
- Vergleichen des proCOL11A1-Protein-Expressionsniveaus in der Probe mit dem proCOL11A1-Protein-Expressionsniveau in einer Kontrollprobe;
wobei die Behandlung ausgewählt wird aus einer pharmakologischen Behandlung, einer Behandlung umfassend die chirurgische Entfernung der papillären Mammaläsion, und einer Kombination davon,
wobei die Behandlung ausgewählt wird:
- wenn das Vorhandensein des proCOL11A1-Proteins in der Probe von der Person, bei der eine papilläre Mammaläsion diagnostiziert wurde, nachgewiesen wird, oder
- wenn das proCOL11A1-Protein-Expressionsniveau in der Probe von der Person, bei der eine papilläre Mammaläsion diagnostiziert wurde, größer ist als das proCOL11A1-Protein-Expressionsniveau in der Kontrollprobe.

16. Das Verfahren gemäß Anspruch 15, wobei die pharmakologische Behandlung die Verabreichung eines Arzneimittels ausgewählt aus der Gruppe bestehend aus Docetaxel, Doxorubicin, 5-Fluoruracil, Methotrexat, Cyclophosphamid, oder deren Kombinationen umfasst.

17. Das Verfahren gemäß Anspruch 15 oder 16, wobei die Behandlung weiterhin nachfolgende Strahlentherapiebehandlung umfasst.

## Revendications

1. Procédé *in vitro* pour pronostiquer l'évolution clinique et/ou prédire le risque de récidive d'une lésion papillaire du sein, sélectionné parmi le procédé (A) et le procédé (B), dans lequel
A) le procédé (A) comprend
- la détection de la présence d'une protéine proCOL11A1 dans un échantillon d'un sujet présentant un diagnostic de lésion papillaire du sein ;
où
- la détection de la présence d'une protéine proCOL11A1 dans ledit échantillon d'un sujet présentant un diagnostic de lésion papillaire du sein indique que le risque de récidive est élevé, ou
- la non-détection de la présence d'une protéine proCOL11A1 dans ledit échantillon d'un sujet présentant un diagnostic de lésion papillaire du sein indique que le risque de récidive est faible, et
B) le procédé (B) comprend
- la comparaison du taux d'expression de la protéine proCOL11A1 dans ledit échantillon avec ledit taux d'expression de la protéine proCOL11A1 dans un échantillon de contrôle;
où
- un taux d'expression de la protéine proCOL11A1 dans ledit échantillon d'un sujet présentant un diagnostic de lésion papillaire du sein plus élevé que ledit taux d'expression de la protéine proCOL11A1 dans un échantillon de contrôle indique que le risque de récidive est élevé, ou
- un taux d'expression de la protéine proCOL11A1 dans ledit échantillon d'un sujet présentant un diagnostic de lésion papillaire du sein équivalent à ou moins élevé que ledit taux d'expression de la protéine proCOL11A1 dans un échantillon de contrôle indique que le risque de récidive est faible.

2. Procédé selon la revendication 1, dans lequel ledit échantillon est un échantillon de tissu mammaire ou un échantillon de liquide biologique.

3. Procédé selon l'une quelconque des revendications 1 ou 2, qui comprend en outre l'obtention, à partir dudit échantillon, d'un extrait protéique ou d'un extrait comprenant de l'ARN total.

4. Procédé selon l'une quelconque des revendications 1 à 3, dans lequel la détection de la protéine proCOL11A1 comprend la mise en contact du tissu mammaire, ou de l'extrait protéique de l'échantillon, avec un anticorps spécifique pour la protéine proCOL11A1, dans des conditions qui permettent la formation d'un complexe anticorps-protéine proCOL11A1.

5. Procédé selon la revendication 4, dans lequel ledit anticorps spécifique pour la protéine proCOL11A1 est un anticorps qui reconnaît un épitope localisé dans le domaine VAR de l'extrémité N-terminale de la protéine proCOL11A1.

6. Procédé selon la revendication 4 ou 5, dans lequel ledit anticorps spécifique pour la protéine proCOL11A1 est l'anticorps monoclonal 1E8.33.

7. Procédé selon l'une quelconque des revendications 4 à 6, dans lequel la détection et/ou la quantification du complexe anticorps-protéine proCOL11A1 formé est réalisée au moyen d'une technique sélectionnée dans le groupe consistant en un transfert de Western, un ELISA, un RIA, un EIA compétitif, un DAS-ELISA, des techniques d'immunocytochimie et d'immunohistochimie, des techniques de détection multiplexe basées sur l'utilisation de microsphères protéiques, des biopuces ou des microréseaux comprenant des anticorps spécifiques, ou des essais basés sur une précipitation colloïdale.

8. Procédé selon l'une quelconque des revendications précédentes, dans lequel ladite récidive est une récidive d'un carcinome du sein ou d'une tumeur du sein.

9. Procédé selon l'une quelconque des revendications précédentes, dans lequel ladite récidive est une récidive d'un adénocarcinome canalaire, un carcinome canalaire *in situ*, un carcinome canalaire infiltrant, un carcinome lobulaire, un carcinome lobulaire *in situ*, un carcinome lobulaire infiltrant, un carcinome tubulaire, un carcinome médullaire, un carcinome mucineux ou colloïde, un carcinome papillaire, un carcinome inflammatoire, un carcinome cribriforme, la maladie de Paget du sein, le cancer du sein infiltrant ou le cancer du sein métastatique.

10. Procédé selon l'une quelconque des revendications précédentes, dans lequel ladite lésion papillaire du sein est un papillome intracanalaire du sein.

11. Procédé pour sélectionner un sujet présentant un diagnostic de lésion papillaire du sein pour un traitement, comprenant l'exérèse chirurgicale de ladite lésion papillaire du sein au moyen d'un procédé qui permet de garantir une exérèse complète de la lésion et d'un traitement pharmacologique préventif pour prévenir toute récidive, ainsi qu'un suivi complet de l'évolution de la lésion, qui permet une détection précoce de l'éventuelle récidive, qui comprend
- la détection de la présence d'une protéine proCOL11A1 dans un échantillon d'un sujet présentant un diagnostic de lésion papillaire du sein ; ou
- la comparaison du taux d'expression de la protéine proCOL11A1 dans ledit échantillon avec ledit taux d'expression de la protéine proCOL11A1 dans un échantillon de contrôle ;
où ledit sujet est sélectionné pour ledit traitement :
- si la présence de la protéine proCOL11A1 est détectée dans ledit échantillon du sujet présentant un diagnostic de lésion papillaire du sein ; ou
- si le taux d'expression de la protéine proCOL11A1 dans ledit échantillon du sujet présentant un diagnostic de lésion papillaire du sein est plus élevé que ledit taux d'expression de la protéine proCOL11A1 dans l'échantillon de contrôle.

12. Procédé selon la revendication 11, dans lequel ledit traitement comprenant l'exérèse chirurgicale de ladite lésion papillaire du sein au moyen d'un procédé qui permet de garantir une exérèse complète de la lésion comprend la réalisation d'une mastectomie partielle ou totale.

13. Procédé pour sélectionner un sujet présentant un diagnostic de lésion papillaire du sein pour un traitement, sélectionné parmi un traitement comprenant l'exérèse de ladite lésion papillaire du sein par une chirurgie conservatrice et un traitement comprenant la non-exérèse de ladite lésion papillaire du sein, qui comprend
- la détection de la présence d'une protéine proCOL11A1 dans un échantillon d'un sujet présentant un diagnostic de lésion papillaire du sein ; ou
- la comparaison du taux d'expression de la protéine proCOL11A1 dans ledit échantillon avec ledit taux d'expression de la protéine proCOL11A1 dans un échantillon de contrôle ;
où ledit sujet est sélectionné pour ledit traitement :
- si la présence de la protéine proCOL11A1 n'est pas détectée dans ledit échantillon du sujet présentant un diagnostic de lésion papillaire du sein ; ou
- si le taux d'expression de la protéine proCOL11A1 dans ledit échantillon du sujet présentant un diagnostic de lésion papillaire du sein est équivalent à ou moins élevé que ledit taux d'expression de la protéine proCOL11A1 dans l'échantillon de contrôle.

14. Procédé selon la revendication 13, dans lequel ledit traitement comprenant l'exérèse de la lésion papillaire du sein par une chirurgie conservatrice comprend une résection au microtome, une résection de ladite lésion papillaire du sein par des prélèvements, ou une résection en bloc comprenant le canal affecté.

15. Procédé pour sélectionner un traitement pour un sujet présentant un diagnostic de lésion papillaire du sein, qui comprend
- la détection de la présence d'une protéine proCOL11A1 dans un échantillon d'un sujet présentant un diagnostic de lésion papillaire du sein ; ou
- la comparaison du taux d'expression de la protéine proCOL11A1 dans ledit échantillon avec ledit taux d'expression de la protéine proCOL11A1 dans un échantillon de contrôle ;
dans lequel ledit traitement est sélectionné parmi un traitement pharmacologique, un traitement comprenant une exérèse chirurgicale de ladite lésion papillaire du sein, et une combinaison de ceux-ci,
où ledit traitement est sélectionné :
- si la présence de la protéine proCOL11A1 est détectée dans ledit échantillon du sujet présentant un diagnostic de lésion papillaire du sein ; ou
- si le taux d'expression de la protéine proCOL11A1 dans ledit échantillon du sujet présentant un diagnostic de lésion papillaire du sein est plus élevé que ledit taux d'expression de la protéine proCOL11A1 dans l'échantillon de contrôle.

16. Procédé selon la revendication 15, dans lequel ledit traitement pharmacologique comprend l'administration d'un médicament sélectionné dans le groupe consistant en le docétaxel, la doxorubicine, le 5-fluorouracile, le méthotrexate, le cyclophosphamide, ou leurs combinaisons.

17. Procédé selon la revendication 15 ou 16, dans lequel ledit traitement comprend en outre un traitement de radiothérapie ultérieur.
